# EUROPEAN PATENT SPECIFICATION

(11) **EP 4 247 278 B1**
(45) Date of publication and mention of the grant of the patent: **20.11.2024**
(21) Application number: 21816162.8
(22) Date of filing: 19.11.2021
(51) Int. Cl.: A61B 17/34, A61B 34/30, A61B 90/00, A61B 90/92, A61B 90/94

(54) **REMOTE CENTERS AND STABILITY SYSTEMS FOR ROBOTIC MEDICAL SYSTEMS**
FERNZENTREN UND STABILITÄTSSYSTEME FÜR ROBOTERMEDIZINISCHE SYSTEME
CENTRES À DISTANCE ET SYSTÈMES DE STABILITÉ POUR SYSTÈMES MÉDICAUX ROBOTIQUES

(30) Priority: 19.11.2020 US 202063116143 P
(43) Date of publication of application: 27.09.2023
(73) Proprietor: Cilag GmbH International, 6300 Zug (CH)
(72) Inventor: CROSETTI, Marissa Anne, San Francisco, California 94109 (US); HASSAN, Alexander Tarek, San Francisco, California 94133 (US); SCHEUNERT, Mary Margaret, San Francisco, California 94103 (US); STONE, Christopher Brock, Redwood City, California 94065 (US); MOZLOOM, JR., Joseph Thomas, Blue Ash, Ohio 45242 (US)
(74) Representative: Carpmaels & Ransford LLP
(86) International application number: PCT/IB2021/060745
(87) International publication number: WO 2022/107061

(56) References cited:
- EP-A1- 1 738 701
- WO-A2-2020/130558
- CA-A1- 3 123 233
- DE-T2- 60 006 942
- DE-U1- 9 014 195
- US-A1- 2018 021 061
- US-A1- 2019 000 505
- US-A1- 2020 315 721

## Description

### TECHNICAL FIELD

Systems disclosed herein are related to medical devices, and more particularly to remote centers and stability systems for robotic medical systems.

### BACKGROUND

Minimally invasive procedures allow for access to a targeted site within a patient with minimal trauma to the patient. For example, laparoscopic surgery can allow for surgical access to a patient's cavity through a small incision on the patient's abdomen. A cannula can form a surgical corridor to allow elongate medical instruments to access the internal anatomical site to perform a procedural function such as manipulating or visualizing tissue within the patient. During operation, the cannula and the instrument can be pivoted about a remote center of motion defined at the intersection with the patient body wall, in order to provide access to various areas within the patient cavity without exerting undue stresses on the patient.

US2018/021061A1 describes a cannula including a cannula bowl having an opening to receive an instrument configured to be advanced through the cannula, and a cannula tube extending distally from the cannula bowl. The cannula tube may have a proximal end opening, a distal end opening disposed at an opposite end of the cannula from the proximal end opening, and lateral wall defining a passage extending from the proximal end opening to the distal end opening. The lateral wall can have outer dimensions defining a waisted portion with smaller outer dimensions than a region disposed proximally or distally to the waisted portion along a length of the cannula tube. The cannula includes radially protruding ribs extending from an outer lateral wall surface. DE60006942T2 describes a cannula having a proximal and distal series of external fixation elements displaced by a certain distance which provides a central tissue receiving area that receives the proximal and distal surfaces of the tissue. Further prior art is disclosed in US 2020/315721 A1, EP 1 738 701 A1, CA 3 123 233 A1, US 2019/000505 A1, DE 90 14 195 U1, WO 2020/130558 A2.

### BRIEF DESCRIPTION OF THE DRAWINGS

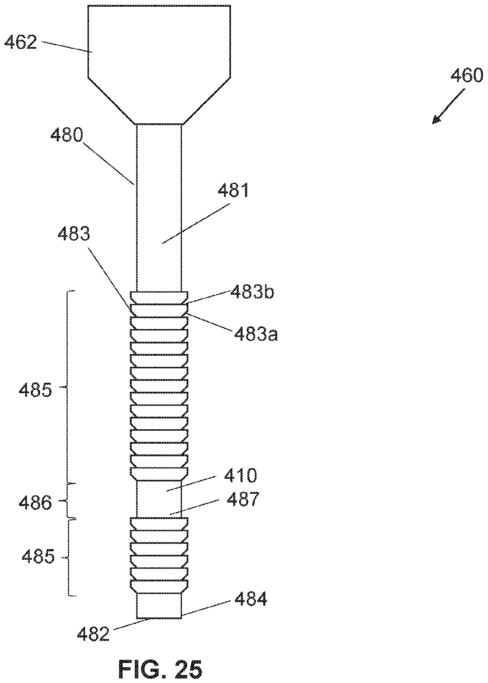

The invention is as defined in claim 1. Figs. 25-28 illustrate some exemplary embodiments of the invention.

The disclosed aspects will hereinafter be described in conjunction with the appended drawings, provided to illustrate and not to limit the disclosed aspects, wherein like designations denote like elements
FIG. 1 illustrates an embodiment of a cart-based robotic system arranged for diagnostic and/or therapeutic bronchoscopy procedure(s).
FIG. 2 depicts further aspects of the robotic system of FIG. 1.
FIG. 3 illustrates an embodiment of the robotic system of FIG. 1 arranged for ureteroscopy.
FIG. 4 illustrates an embodiment of the robotic system of FIG. 1 arranged for a vascular procedure.
FIG. 5 illustrates an embodiment of a table-based robotic system arranged for a bronchoscopy procedure.
FIG. 6 provides an alternative view of the robotic system of FIG. 5.
FIG. 7 illustrates an example system configured to stow robotic arm(s).
FIG. 8 illustrates an embodiment of a table-based robotic system configured for a ureteroscopy procedure.
FIG. 9 illustrates an embodiment of a table-based robotic system configured for a laparoscopic procedure.
FIG. 10 illustrates an embodiment of the table-based robotic system of FIGS. 5-9 with pitch or tilt adjustment.
FIG. 11 provides a detailed illustration of the interface between the table and the column of the table-based robotic system of FIGS. 5-10.
FIG. 12 illustrates an alternative embodiment of a table-based robotic system.
FIG. 13 illustrates an end view of the table-based robotic system of FIG. 12.
FIG. 14 illustrates an end view of a table-based robotic system with robotic arms attached thereto.
FIG. 15 illustrates an exemplary instrument driver.
FIG. 16 illustrates an exemplary medical instrument with a paired instrument driver.
FIG. 17 illustrates an alternative design for an instrument driver and instrument where the axes of the drive units are parallel to the axis of the elongated shaft of the instrument.
FIG. 18 illustrates an instrument having an instrument-based insertion architecture.
FIG. 19 illustrates an exemplary controller.
FIG. 20 depicts a block diagram illustrating a localization system that estimates a location of one or more elements of the robotic systems of FIGS. 1-10, such as the location of the instrument of FIGS. 16-18, in accordance to an example embodiment.
FIG. 21 illustrates an exemplary cannula.
FIG. 22 illustrates an exemplary docking arrangement.
FIG. 23 illustrates an exemplary cannula pivoted about a remote center of motion.
FIG. 24 illustrates an exemplary misaligned cannula.
FIG. 25 illustrates an exemplary cannula having ridges and a defined remote center.
FIG. 26 illustrates an exemplary cannula installed in a body wall.
FIG. 27 illustrates an exemplary sequence of switching between remote centers.
FIG. 28 illustrates an exemplary cannula having ridges and multiple remote centers.
FIG. 29 illustrates an exemplary cannula having multiple indicators for multiple remote centers.
FIG. 30 illustrates an exemplary cannula having multiple indicators for multiple remote centers.
FIG. 31 illustrates an exemplary cannula having multiple indicators for multiple remote centers.
FIG. 32 illustrates an exemplary cannula having multiple indicators for multiple remote centers.
FIG. 33 illustrates an exemplary cannula having multiple indicators for multiple remote centers.

### DETAILED DESCRIPTION

### 1. Overview.

Aspects of the present disclosure may be integrated into a robotically-enabled medical system capable of performing a variety of medical procedures, including both minimally invasive, such as laparoscopy, and non-invasive, such as endoscopy, procedures. Among endoscopy procedures, the system may be capable of performing bronchoscopy, ureteroscopy, gastroscopy, etc.

In addition to performing the breadth of procedures, the system may provide additional benefits, such as enhanced imaging and guidance to assist the physician. Additionally, the system may provide the physician with the ability to perform the procedure from an ergonomic position without the need for awkward arm motions and positions. Still further, the system may provide the physician with the ability to perform the procedure with improved ease of use such that one or more of the instruments of the system can be controlled by a single user.

Various embodiments will be described below in conjunction with the drawings for purposes of illustration. It should be appreciated that many other implementations of the disclosed concepts are possible, and various advantages can be achieved with the disclosed implementations. Headings are included herein for reference and to aid in locating various sections. These headings are not intended to limit the scope of the concepts described with respect thereto. Such concepts may have applicability throughout the entire specification.

### A. Robotic System - Cart.

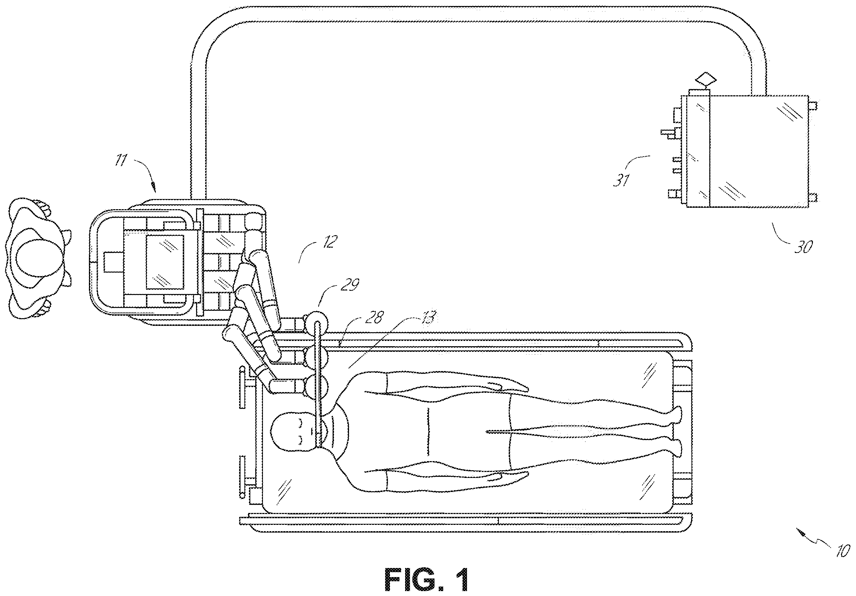

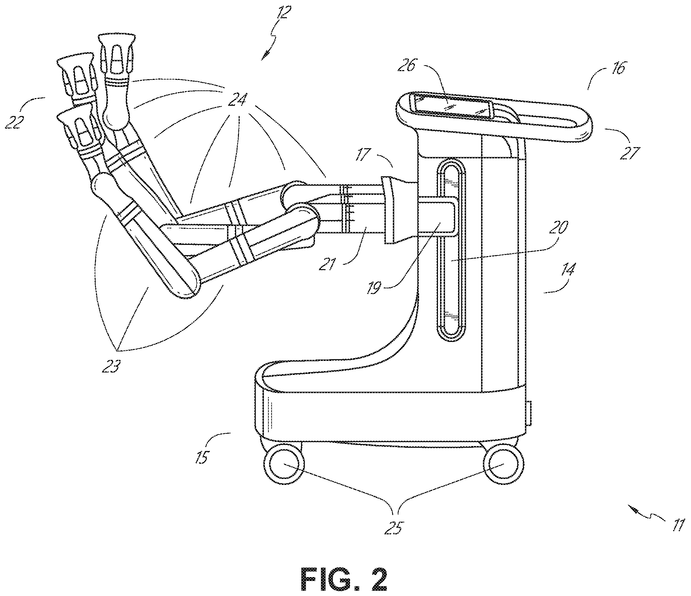

The robotically-enabled medical system may be configured in a variety of ways depending on the particular procedure. FIG. 1 illustrates an embodiment of a cart-based robotically-enabled system 10 arranged for a diagnostic and/or therapeutic bronchoscopy procedure. During a bronchoscopy, the system 10 may comprise a cart 11 having one or more robotic arms 12 to deliver a medical instrument, such as a steerable endoscope 13, which may be a procedure-specific bronchoscope for bronchoscopy, to a natural orifice access point (i.e., the mouth of the patient positioned on a table in the present example) to deliver diagnostic and/or therapeutic tools. As shown, the cart 11 may be positioned proximate to the patient's upper torso in order to provide access to the access point. Similarly, the robotic arms 12 may be actuated to position the bronchoscope relative to the access point. The arrangement in FIG. 1 may also be utilized when performing a gastro-intestinal (GI) procedure with a gastroscope, a specialized endoscope for GI procedures. FIG. 2 depicts an example embodiment of the cart in greater detail.

With continued reference to FIG. 1, once the cart 11 is properly positioned, the robotic arms 12 may insert the steerable endoscope 13 into the patient robotically, manually, or a combination thereof. As shown, the steerable endoscope 13 may comprise at least two telescoping parts, such as an inner leader portion and an outer sheath portion, each portion coupled to a separate instrument driver from the set of instrument drivers 28, each instrument driver coupled to the distal end of an individual robotic arm. This linear arrangement of the instrument drivers 28, which facilitates coaxially aligning the leader portion with the sheath portion, creates a "virtual rail" 29 that may be repositioned in space by manipulating the one or more robotic arms 12 into different angles and/or positions. The virtual rails described herein are depicted in the Figures using dashed lines, and accordingly the dashed lines do not depict any physical structure of the system. Translation of the instrument drivers 28 along the virtual rail 29 telescopes the inner leader portion relative to the outer sheath portion or advances or retracts the endoscope 13 from the patient. The angle of the virtual rail 29 may be adjusted, translated, and pivoted based on clinical application or physician preference. For example, in bronchoscopy, the angle and position of the virtual rail 29 as shown represents a compromise between providing physician access to the endoscope 13 while minimizing friction that results from bending the endoscope 13 into the patient's mouth.

The endoscope 13 may be directed down the patient's trachea and lungs after insertion using precise commands from the robotic system until reaching the target destination or operative site. In order to enhance navigation through the patient's lung network and/or reach the desired target, the endoscope 13 may be manipulated to telescopically extend the inner leader portion from the outer sheath portion to obtain enhanced articulation and greater bend radius. The use of separate instrument drivers 28 also allows the leader portion and sheath portion to be driven independent of each other.

For example, the endoscope 13 may be directed to deliver a biopsy needle to a target, such as, for example, a lesion or nodule within the lungs of a patient. The needle may be deployed down a working channel that runs the length of the endoscope to obtain a tissue sample to be analyzed by a pathologist. Depending on the pathology results, additional tools may be deployed down the working channel of the endoscope for additional biopsies. After identifying a nodule to be malignant, the endoscope 13 may endoscopically deliver tools to resect the potentially cancerous tissue. In some instances, diagnostic and therapeutic treatments can be delivered in separate procedures. In those circumstances, the endoscope 13 may also be used to deliver a fiducial to "mark" the location of the target nodule as well. In other instances, diagnostic and therapeutic treatments may be delivered during the same procedure.

The system 10 may also include a movable tower 30, which may be connected via support cables to the cart 11 to provide support for controls, electronics, fluidics, optics, sensors, and/or power to the cart 11. Placing such functionality in the tower 30 allows for a smaller form factor cart 11 that may be more easily adjusted and/or re-positioned by an operating physician and his/her staff. Additionally, the division of functionality between the cart / table and the support tower 30 reduces operating room clutter and facilitates improving clinical workflow. While the cart 11 may be positioned close to the patient, the tower 30 may be stowed in a remote location to stay out of the way during a procedure.

In support of the robotic systems described above, the tower 30 may include component(s) of a computer-based control system that stores computer program instructions, for example, within a non-transitory computer-readable storage medium such as a persistent magnetic storage drive, solid state drive, etc. The execution of those instructions, whether the execution occurs in the tower 30 or the cart 11, may control the entire system or sub-system(s) thereof. For example, when executed by a processor of the computer system, the instructions may cause the components of the robotics system to actuate the relevant carriages and arm mounts, actuate the robotics arms, and control the medical instruments. For example, in response to receiving the control signal, the motors in the joints of the robotics arms may position the arms into a certain posture.

The tower 30 may also include a pump, flow meter, valve control, and/or fluid access in order to provide controlled irrigation and aspiration capabilities to the system that may be deployed through the endoscope 13. These components may also be controlled using the computer system of tower 30. In some embodiments, irrigation and aspiration capabilities may be delivered directly to the endoscope 13 through separate cable(s).

The tower 30 may include a voltage and surge protector designed to provide filtered and protected electrical power to the cart 11, thereby avoiding placement of a power transformer and other auxiliary power components in the cart 11, resulting in a smaller, more moveable cart 11.

The tower 30 may also include support equipment for the sensors deployed throughout the robotic system 10. For example, the tower 30 may include opto-electronics equipment for detecting, receiving, and processing data received from the optical sensors or cameras throughout the robotic system 10. In combination with the control system, such opto-electronics equipment may be used to generate real-time images for display in any number of consoles deployed throughout the system, including in the tower 30. Similarly, the tower 30 may also include an electronic subsystem for receiving and processing signals received from deployed electromagnetic (EM) sensors. The tower 30 may also be used to house and position an EM field generator for detection by EM sensors in or on the medical instrument.

The tower 30 may also include a console 31 in addition to other consoles available in the rest of the system, e.g., console mounted on top of the cart. The console 31 may include a user interface and a display screen, such as a touchscreen, for the physician operator. Consoles in system 10 are generally designed to provide both robotic controls as well as pre-operative and real-time information of the procedure, such as navigational and localization information of the endoscope 13. When the console 31 is not the only console available to the physician, it may be used by a second operator, such as a nurse, to monitor the health or vitals of the patient and the operation of system, as well as provide procedure-specific data, such as navigational and localization information. In other embodiments, the console 30 is housed in a body that is separate from the tower 30.

The tower 30 may be coupled to the cart 11 and endoscope 13 through one or more cables or connections (not shown). In some embodiments, the support functionality from the tower 30 may be provided through a single cable to the cart 11, simplifying and de-cluttering the operating room. In other embodiments, specific functionality may be coupled in separate cabling and connections. For example, while power may be provided through a single power cable to the cart, the support for controls, optics, fluidics, and/or navigation may be provided through a separate cable.

FIG. 2 provides a detailed illustration of an embodiment of the cart from the cart-based robotically-enabled system shown in FIG. 1. The cart 11 generally includes an elongated support structure 14 (often referred to as a "column"), a cart base 15, and a console 16 at the top of the column 14. The column 14 may include one or more carriages, such as a carriage 17 (alternatively "arm support") for supporting the deployment of one or more robotic arms 12 (three shown in FIG. 2). The carriage 17 may include individually configurable arm mounts that rotate along a perpendicular axis to adjust the base of the robotic arms 12 for better positioning relative to the patient. The carriage 17 also includes a carriage interface 19 that allows the carriage 17 to vertically translate along the column 14.

The carriage interface 19 is connected to the column 14 through slots, such as slot 20, that are positioned on opposite sides of the column 14 to guide the vertical translation of the carriage 17. The slot 20 contains a vertical translation interface to position and hold the carriage at various vertical heights relative to the cart base 15. Vertical translation of the carriage 17 allows the cart 11 to adjust the reach of the robotic arms 12 to meet a variety of table heights, patient sizes, and physician preferences. Similarly, the individually configurable arm mounts on the carriage 17 allow the robotic arm base 21 of robotic arms 12 to be angled in a variety of configurations.

In some embodiments, the slot 20 may be supplemented with slot covers that are flush and parallel to the slot surface to prevent dirt and fluid ingress into the internal chambers of the column 14 and the vertical translation interface as the carriage 17 vertically translates. The slot covers may be deployed through pairs of spring spools positioned near the vertical top and bottom of the slot 20. The covers are coiled within the spools until deployed to extend and retract from their coiled state as the carriage 17 vertically translates up and down. The spring-loading of the spools provides force to retract the cover into a spool when carriage 17 translates towards the spool, while also maintaining a tight seal when the carriage 17 translates away from the spool. The covers may be connected to the carriage 17 using, for example, brackets in the carriage interface 19 to ensure proper extension and retraction of the cover as the carriage 17 translates.

The column 14 may internally comprise mechanisms, such as gears and motors, that are designed to use a vertically aligned lead screw to translate the carriage 17 in a mechanized fashion in response to control signals generated in response to user inputs, e.g., inputs from the console 16.

The robotic arms 12 may generally comprise robotic arm bases 21 and end effectors 22, separated by a series of linkages 23 that are connected by a series of joints 24, each joint comprising an independent actuator, each actuator comprising an independently controllable motor. Each independently controllable joint represents an independent degree of freedom available to the robotic arm. Each of the arms 12 have seven joints, and thus provide seven degrees of freedom. A multitude of joints result in a multitude of degrees of freedom, allowing for "redundant" degrees of freedom. Redundant degrees of freedom allow the robotic arms 12 to position their respective end effectors 22 at a specific position, orientation, and trajectory in space using different linkage positions and joint angles. This allows for the system to position and direct a medical instrument from a desired point in space while allowing the physician to move the arm joints into a clinically advantageous position away from the patient to create greater access, while avoiding arm collisions.

The cart base 15 balances the weight of the column 14, carriage 17, and arms 12 over the floor. Accordingly, the cart base 15 houses heavier components, such as electronics, motors, power supply, as well as components that either enable movement and/or immobilize the cart. For example, the cart base 15 includes rollable wheel-shaped casters 25 that allow for the cart to easily move around the room prior to a procedure. After reaching the appropriate position, the casters 25 may be immobilized using wheel locks to hold the cart 11 in place during the procedure.

Positioned at the vertical end of column 14, the console 16 allows for both a user interface for receiving user input and a display screen (or a dual-purpose device such as, for example, a touchscreen 26) to provide the physician user with both pre-operative and intra-operative data. Potential pre-operative data on the touchscreen 26 may include pre-operative plans, navigation and mapping data derived from pre-operative computerized tomography (CT) scans, and/or notes from pre-operative patient interviews. Intra-operative data on display may include optical information provided from the tool, sensor and coordinate information from sensors, as well as vital patient statistics, such as respiration, heart rate, and/or pulse. The console 16 may be positioned and tilted to allow a physician to access the console from the side of the column 14 opposite carriage 17. From this position, the physician may view the console 16, robotic arms 12, and patient while operating the console 16 from behind the cart 11. As shown, the console 16 also includes a handle 27 to assist with maneuvering and stabilizing cart 11.

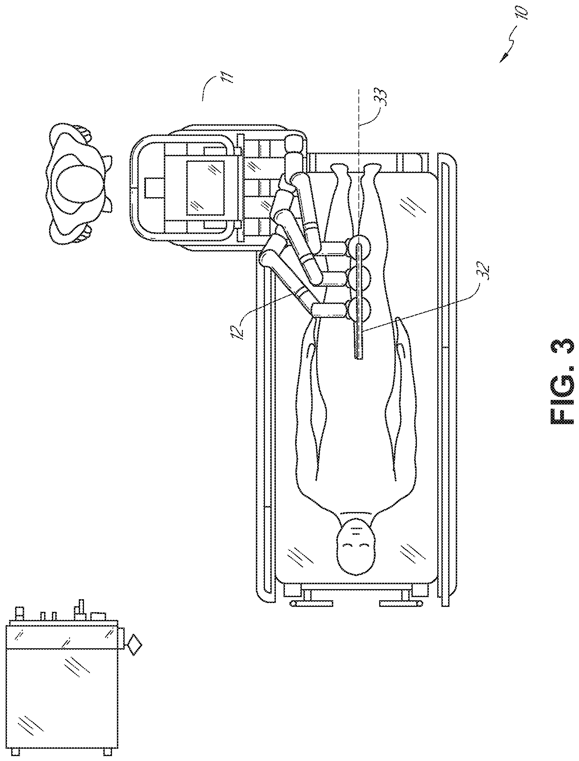

FIG. 3 illustrates an embodiment of a robotically-enabled system 10 arranged for ureteroscopy. In a ureteroscopic procedure, the cart 11 may be positioned to deliver a ureteroscope 32, a procedure-specific endoscope designed to traverse a patient's urethra and ureter, to the lower abdominal area of the patient. In a ureteroscopy, it may be desirable for the ureteroscope 32 to be directly aligned with the patient's urethra to reduce friction and forces on the sensitive anatomy in the area. As shown, the cart 11 may be aligned at the foot of the table to allow the robotic arms 12 to position the ureteroscope 32 for direct linear access to the patient's urethra. From the foot of the table, the robotic arms 12 may insert the ureteroscope 32 along the virtual rail 33 directly into the patient's lower abdomen through the urethra.

After insertion into the urethra, using similar control techniques as in bronchoscopy, the ureteroscope 32 may be navigated into the bladder, ureters, and/or kidneys for diagnostic and/or therapeutic applications. For example, the ureteroscope 32 may be directed into the ureter and kidneys to break up kidney stone build up using a laser or ultrasonic lithotripsy device deployed down the working channel of the ureteroscope 32. After lithotripsy is complete, the resulting stone fragments may be removed using baskets deployed down the ureteroscope 32.

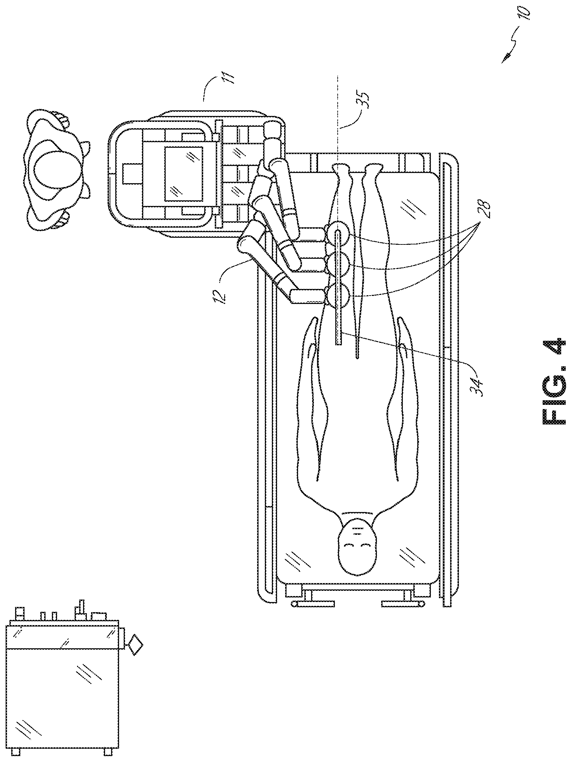

FIG. 4 illustrates an embodiment of a robotically-enabled system similarly arranged for a vascular procedure. In a vascular procedure, the system 10 may be configured such that the cart 11 may deliver a medical instrument 34, such as a steerable catheter, to an access point in the femoral artery in the patient's leg. The femoral artery presents both a larger diameter for navigation as well as a relatively less circuitous and tortuous path to the patient's heart, which simplifies navigation. As in a ureteroscopic procedure, the cart 11 may be positioned towards the patient's legs and lower abdomen to allow the robotic arms 12 to provide a virtual rail 35 with direct linear access to the femoral artery access point in the patient's thigh / hip region. After insertion into the artery, the medical instrument 34 may be directed and inserted by translating the instrument drivers 28. Alternatively, the cart may be positioned around the patient's upper abdomen in order to reach alternative vascular access points, such as, for example, the carotid and brachial arteries near the shoulder and wrist.

### B. Robotic System - Table.

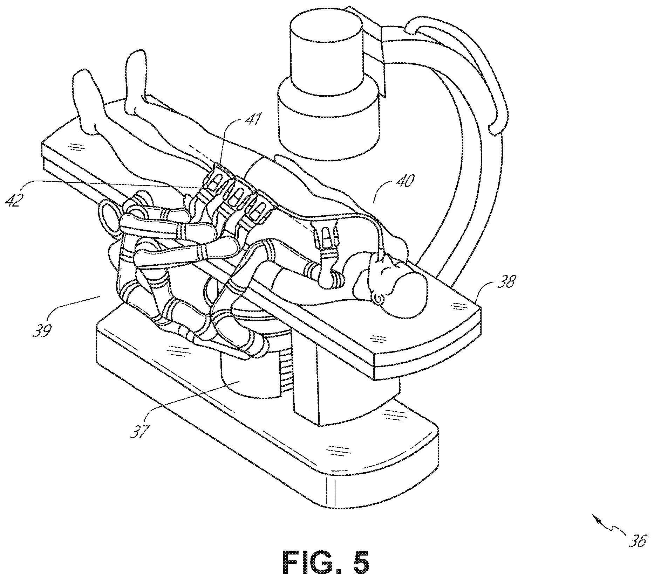

Embodiments of the robotically-enabled medical system may also incorporate the patient's table. Incorporation of the table reduces the amount of capital equipment within the operating room by removing the cart, which allows greater access to the patient. FIG. 5 illustrates an embodiment of such a robotically-enabled system arranged for a bronchoscopy procedure. System 36 includes a support structure or column 37 for supporting platform 38 (shown as a "table" or "bed") over the floor. Much like in the cart-based systems, the end effectors of the robotic arms 39 of the system 36 comprise instrument drivers 42 that are designed to manipulate an elongated medical instrument, such as a bronchoscope 40 in FIG. 5, through or along a virtual rail 41 formed from the linear alignment of the instrument drivers 42. In practice, a C-arm for providing fluoroscopic imaging may be positioned over the patient's upper abdominal area by placing the emitter and detector around table 38.

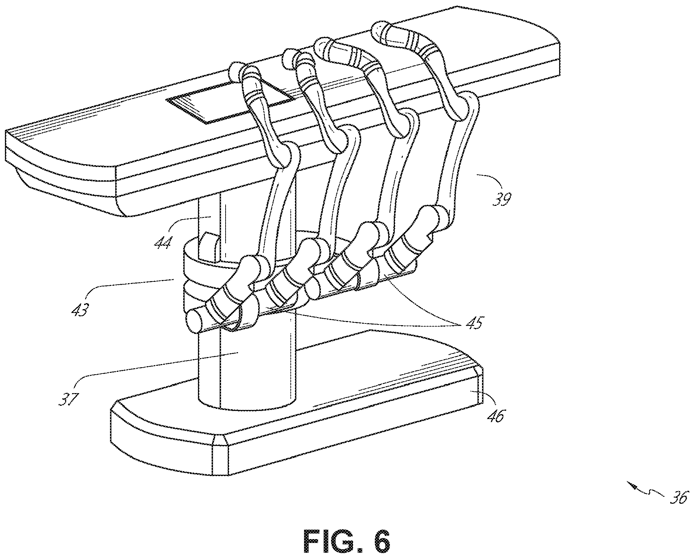

FIG. 6 provides an alternative view of the system 36 without the patient and medical instrument for discussion purposes. As shown, the column 37 may include one or more carriages 43 shown as ring-shaped in the system 36, from which the one or more robotic arms 39 may be based. The carriages 43 may translate along a vertical column interface 44 that runs the length of the column 37 to provide different vantage points from which the robotic arms 39 may be positioned to reach the patient. The carriage(s) 43 may rotate around the column 37 using a mechanical motor positioned within the column 37 to allow the robotic arms 39 to have access to multiples sides of the table 38, such as, for example, both sides of the patient. In embodiments with multiple carriages, the carriages may be individually positioned on the column and may translate and/or rotate independent of the other carriages. While carriages 43 need not surround the column 37 or even be circular, the ring-shape as shown facilitates rotation of the carriages 43 around the column 37 while maintaining structural balance. Rotation and translation of the carriages 43 allows the system to align the medical instruments, such as endoscopes and laparoscopes, into different access points on the patient. In other embodiments (not shown), the system 36 can include a patient table or bed with adjustable arm supports in the form of bars or rails extending alongside it. One or more robotic arms 39 (e.g., via a shoulder with an elbow joint) can be attached to the adjustable arm supports, which can be vertically adjusted. By providing vertical adjustment, the robotic arms 39 are advantageously capable of being stowed compactly beneath the patient table or bed, and subsequently raised during a procedure.

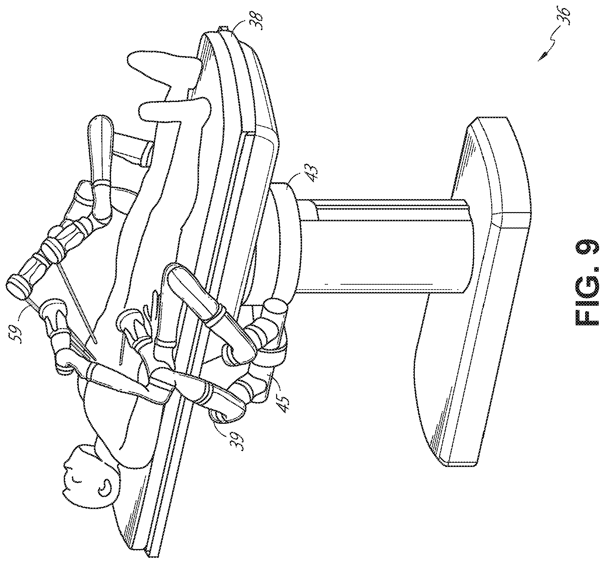

The arms 39 may be mounted on the carriages through a set of arm mounts 45 comprising a series of joints that may individually rotate and/or telescopically extend to provide additional configurability to the robotic arms 39. Additionally, the arm mounts 45 may be positioned on the carriages 43 such that, when the carriages 43 are appropriately rotated, the arm mounts 45 may be positioned on either the same side of table 38 (as shown in FIG. 6), on opposite sides of table 38 (as shown in FIG. 9), or on adjacent sides of the table 38 (not shown).

The column 37 structurally provides support for the table 38, and a path for vertical translation of the carriages. Internally, the column 37 may be equipped with lead screws for guiding vertical translation of the carriages, and motors to mechanize the translation of said carriages based the lead screws. The column 37 may also convey power and control signals to the carriage 43 and robotic arms 39 mounted thereon.

The table base 46 serves a similar function as the cart base 15 in cart 11 shown in FIG. 2, housing heavier components to balance the table/bed 38, the column 37, the carriages 43, and the robotic arms 39. The table base 46 may also incorporate rigid casters to provide stability during procedures. Deployed from the bottom of the table base 46, the casters may extend in opposite directions on both sides of the base 46 and retract when the system 36 needs to be moved.

Continuing with FIG. 6, the system 36 may also include a tower (not shown) that divides the functionality of system 36 between table and tower to reduce the form factor and bulk of the table. As in earlier disclosed embodiments, the tower may provide a variety of support functionalities to table, such as processing, computing, and control capabilities, power, fluidics, and/or optical and sensor processing. The tower may also be movable to be positioned away from the patient to improve physician access and de-clutter the operating room. Additionally, placing components in the tower allows for more storage space in the table base for potential stowage of the robotic arms. The tower may also include a master controller or console that provides both a user interface for user input, such as keyboard and/or pendant, as well as a display screen (or touchscreen) for pre-operative and intra-operative information, such as real-time imaging, navigation, and tracking information. In some embodiments, the tower may also contain holders for gas tanks to be used for insufflation.

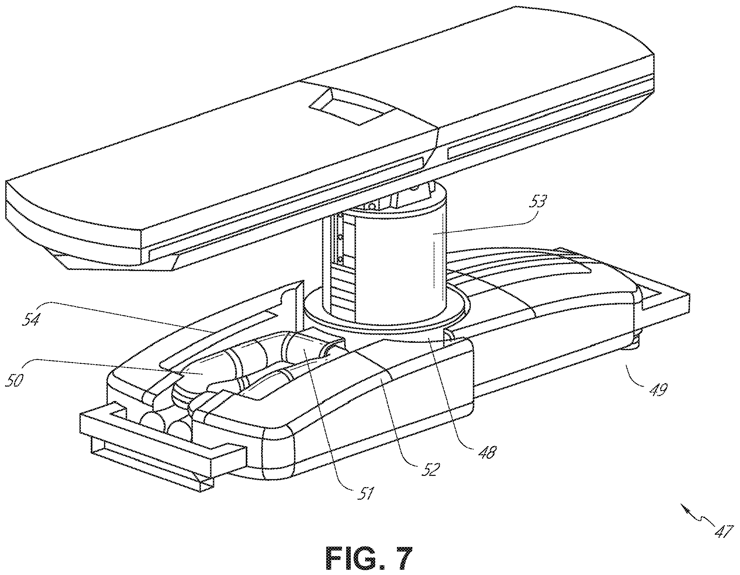

In some embodiments, a table base may stow and store the robotic arms when not in use. FIG. 7 illustrates a system 47 that stows robotic arms in an embodiment of the table-based system. In system 47, carriages 48 may be vertically translated into base 49 to stow robotic arms 50, arm mounts 51, and the carriages 48 within the base 49. Base covers 52 may be translated and retracted open to deploy the carriages 48, arm mounts 51, and arms 50 around column 53, and closed to stow to protect them when not in use. The base covers 52 may be sealed with a membrane 54 along the edges of its opening to prevent dirt and fluid ingress when closed.

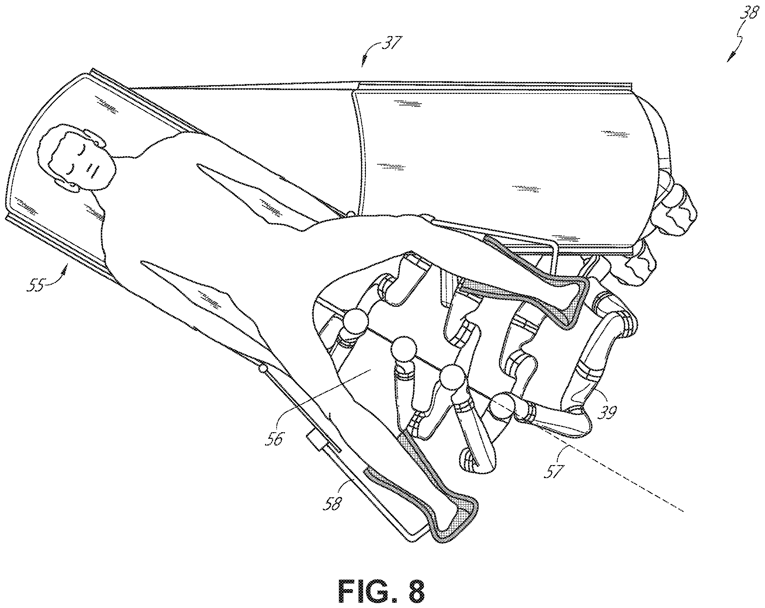

FIG. 8 illustrates an embodiment of a robotically-enabled table-based system configured for a ureteroscopy procedure. In a ureteroscopy, the table 38 may include a swivel portion 55 for positioning a patient off-angle from the column 37 and table base 46. The swivel portion 55 may rotate or pivot around a pivot point (e.g., located below the patient's head) in order to position the bottom portion of the swivel portion 55 away from the column 37. For example, the pivoting of the swivel portion 55 allows a C-arm (not shown) to be positioned over the patient's lower abdomen without competing for space with the column (not shown) below table 38. By rotating the carriage (not shown) around the column 37, the robotic arms 39 may directly insert a ureteroscope 56 along a virtual rail 57 into the patient's groin area to reach the urethra. In a ureteroscopy, stirrups 58 may also be fixed to the swivel portion 55 of the table 38 to support the position of the patient's legs during the procedure and allow clear access to the patient's groin area.

In a laparoscopic procedure, through small incision(s) in the patient's abdominal wall, minimally invasive instruments may be inserted into the patient's anatomy. In some embodiments, the minimally invasive instruments comprise an elongated rigid member, such as a shaft, which is used to access anatomy within the patient. After inflation of the patient's abdominal cavity, the instruments may be directed to perform surgical or medical tasks, such as grasping, cutting, ablating, suturing, etc. In some embodiments, the instruments can comprise a scope, such as a laparoscope. FIG. 9 illustrates an embodiment of a robotically-enabled table-based system configured for a laparoscopic procedure. As shown in FIG. 9, the carriages 43 of the system 36 may be rotated and vertically adjusted to position pairs of the robotic arms 39 on opposite sides of the table 38, such that instrument 59 may be positioned using the arm mounts 45 to be passed through minimal incisions on both sides of the patient to reach his/her abdominal cavity.

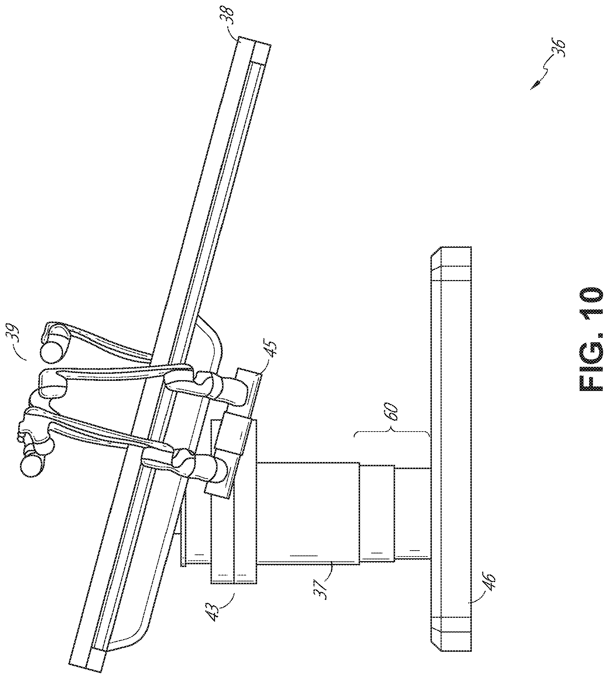

To accommodate laparoscopic procedures, the robotically-enabled table system may also tilt the platform to a desired angle. FIG. 10 illustrates an embodiment of the robotically-enabled medical system with pitch or tilt adjustment. As shown in FIG. 10, the system 36 may accommodate tilt of the table 38 to position one portion of the table at a greater distance from the floor than the other. Additionally, the arm mounts 45 may rotate to match the tilt such that the arms 39 maintain the same planar relationship with table 38. To accommodate steeper angles, the column 37 may also include telescoping portions 60 that allow vertical extension of column 37 to keep the table 38 from touching the floor or colliding with base 46.

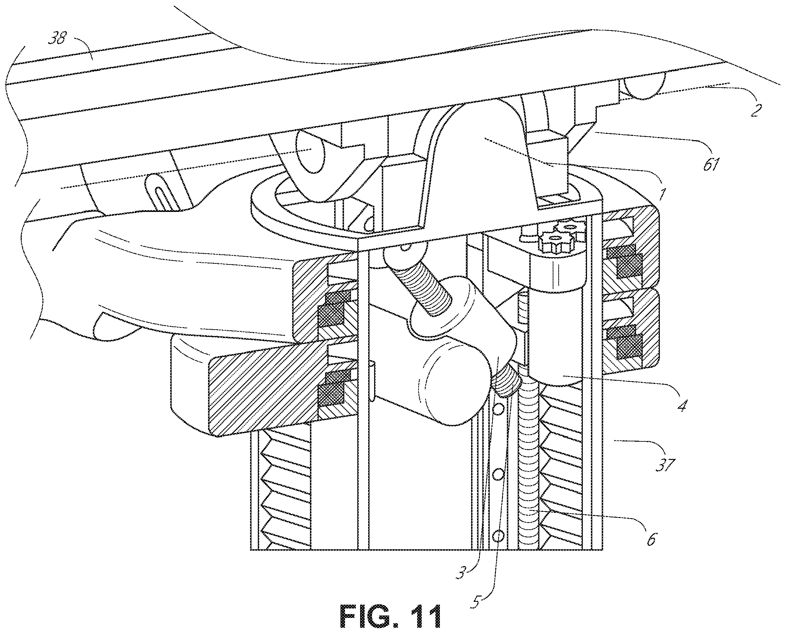

FIG. 11 provides a detailed illustration of the interface between the table 38 and the column 37. Pitch rotation mechanism 61 may be configured to alter the pitch angle of the table 38 relative to the column 37 in multiple degrees of freedom. The pitch rotation mechanism 61 may be enabled by the positioning of orthogonal axes 1, 2 at the column-table interface, each axis actuated by a separate motor 3, 4 responsive to an electrical pitch angle command. Rotation along one screw 5 would enable tilt adjustments in one axis 1, while rotation along the other screw 6 would enable tilt adjustments along the other axis 2. In some embodiments, a ball joint can be used to alter the pitch angle of the table 38 relative to the column 37 in multiple degrees of freedom.

For example, pitch adjustments are particularly useful when trying to position the table in a Trendelenburg position, i.e., position the patient's lower abdomen at a higher position from the floor than the patient's lower abdomen, for lower abdominal surgery. The Trendelenburg position causes the patient's internal organs to slide towards his/her upper abdomen through the force of gravity, clearing out the abdominal cavity for minimally invasive tools to enter and perform lower abdominal surgical or medical procedures, such as laparoscopic prostatectomy.

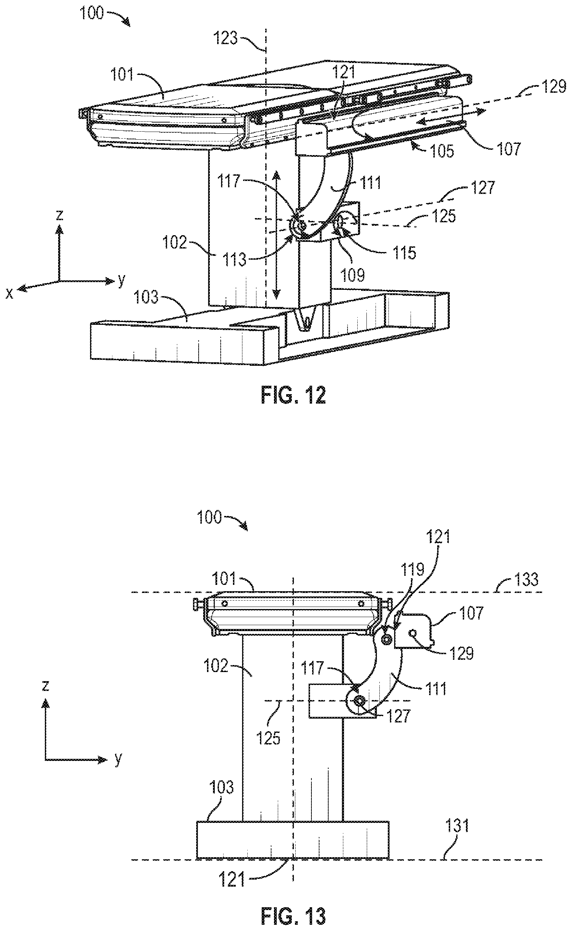

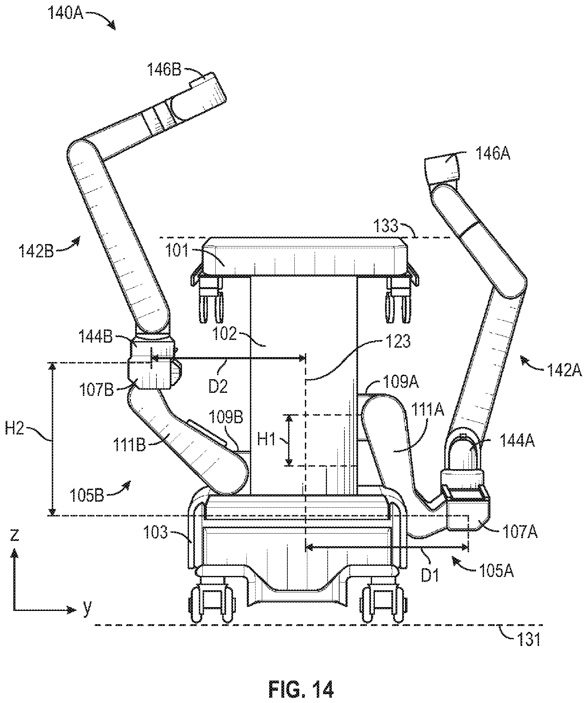

FIGS. 12 and 13 illustrate isometric and end views of an alternative embodiment of a table-based surgical robotics system 100. The surgical robotics system 100 includes one or more adjustable arm supports 105 that can be configured to support one or more robotic arms (see, for example, FIG. 14) relative to a table 101. In the illustrated embodiment, a single adjustable arm support 105 is shown, though an additional arm support can be provided on an opposite side of the table 101. The adjustable arm support 105 can be configured so that it can move relative to the table 101 to adjust and/or vary the position of the adjustable arm support 105 and/or any robotic arms mounted thereto relative to the table 101. For example, the adjustable arm support 105 may be adjusted one or more degrees of freedom relative to the table 101. The adjustable arm support 105 provides high versatility to the system 100, including the ability to easily stow the one or more adjustable arm supports 105 and any robotics arms attached thereto beneath the table 101. The adjustable arm support 105 can be elevated from the stowed position to a position below an upper surface of the table 101. In other embodiments, the adjustable arm support 105 can be elevated from the stowed position to a position above an upper surface of the table 101.

The adjustable arm support 105 can provide several degrees of freedom, including lift, lateral translation, tilt, etc. In the illustrated embodiment of FIGS. 12 and 13, the arm support 105 is configured with four degrees of freedom, which are illustrated with arrows in FIG. 12. A first degree of freedom allows for adjustment of the adjustable arm support 105 in the z-direction ("Z-lift"). For example, the adjustable arm support 105 can include a carriage 109 configured to move up or down along or relative to a column 102 supporting the table 101. A second degree of freedom can allow the adjustable arm support 105 to tilt. For example, the adjustable arm support 105 can include a rotary joint, which can allow the adjustable arm support 105 to be aligned with the bed in a Trendelenburg position. A third degree of freedom can allow the adjustable arm support 105 to "pivot up," which can be used to adjust a distance between a side of the table 101 and the adjustable arm support 105. A fourth degree of freedom can permit translation of the adjustable arm support 105 along a longitudinal length of the table.

The surgical robotics system 100 in FIGS. 12 and 13 can comprise a table supported by a column 102 that is mounted to a base 103. The base 103 and the column 102 support the table 101 relative to a support surface. A floor axis 131 and a support axis 133 are shown in FIG. 13.

The adjustable arm support 105 can be mounted to the column 102. In other embodiments, the arm support 105 can be mounted to the table 101 or base 103. The adjustable arm support 105 can include a carriage 109, a bar or rail connector 111 and a bar or rail 107. In some embodiments, one or more robotic arms mounted to the rail 107 can translate and move relative to one another.

The carriage 109 can be attached to the column 102 by a first joint 113, which allows the carriage 109 to move relative to the column 102 (e.g., such as up and down a first or vertical axis 123). The first joint 113 can provide the first degree of freedom ("Z-lift") to the adjustable arm support 105. The adjustable arm support 105 can include a second joint 115, which provides the second degree of freedom (tilt) for the adjustable arm support 105. The adjustable arm support 105 can include a third joint 117, which can provide the third degree of freedom ("pivot up") for the adjustable arm support 105. An additional joint 119 (shown in FIG. 13) can be provided that mechanically constrains the third joint 117 to maintain an orientation of the rail 107 as the rail connector 111 is rotated about a third axis 127. The adjustable arm support 105 can include a fourth joint 121, which can provide a fourth degree of freedom (translation) for the adjustable arm support 105 along a fourth axis 129.

FIG. 14 illustrates an end view of the surgical robotics system 140A with two adjustable arm supports 105A, 105B mounted on opposite sides of a table 101. A first robotic arm 142A is attached to the bar or rail 107A of the first adjustable arm support 105B. The first robotic arm 142A includes a base 144A attached to the rail 107A. The distal end of the first robotic arm 142A includes an instrument drive mechanism 146A that can attach to one or more robotic medical instruments or tools. Similarly, the second robotic arm 142B includes a base 144B attached to the rail 107B. The distal end of the second robotic arm 142B includes an instrument drive mechanism 146B. The instrument drive mechanism 146B can be configured to attach to one or more robotic medical instruments or tools.

In some embodiments, one or more of the robotic arms 142A, 142B comprises an arm with seven or more degrees of freedom. In some embodiments, one or more of the robotic arms 142A, 142B can include eight degrees of freedom, including an insertion axis (1-degree of freedom including insertion), a wrist (3-degrees of freedom including wrist pitch, yaw and roll), an elbow (1-degree of freedom including elbow pitch), a shoulder (2-degrees of freedom including shoulder pitch and yaw), and base 144A, 144B (1-degree of freedom including translation). In some embodiments, the insertion degree of freedom can be provided by the robotic arm 142A, 142B, while in other embodiments, the instrument itself provides insertion via an instrument-based insertion architecture.

### C. Instrument Driver & Interface.

The end effectors of the system's robotic arms comprise (i) an instrument driver (alternatively referred to as "instrument drive mechanism" or "instrument device manipulator") that incorporate electro-mechanical means for actuating the medical instrument and (ii) a removable or detachable medical instrument, which may be devoid of any electro-mechanical components, such as motors. This dichotomy may be driven by the need to sterilize medical instruments used in medical procedures, and the inability to adequately sterilize expensive capital equipment due to their intricate mechanical assemblies and sensitive electronics. Accordingly, the medical instruments may be designed to be detached, removed, and interchanged from the instrument driver (and thus the system) for individual sterilization or disposal by the physician or the physician's staff. In contrast, the instrument drivers need not be changed or sterilized, and may be draped for protection.

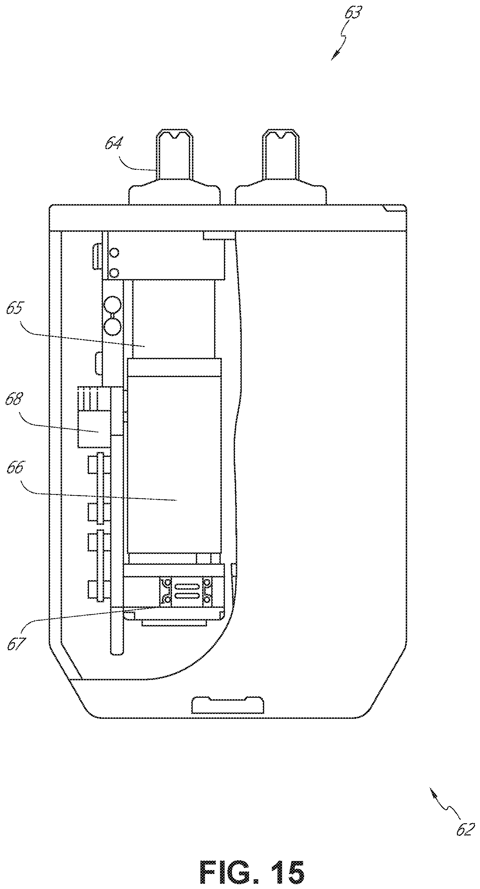

FIG. 15 illustrates an example instrument driver. Positioned at the distal end of a robotic arm, instrument driver 62 comprises of one or more drive units 63 arranged with parallel axes to provide controlled torque to a medical instrument via drive shafts 64. Each drive unit 63 comprises an individual drive shaft 64 for interacting with the instrument, a gear head 65 for converting the motor shaft rotation to a desired torque, a motor 66 for generating the drive torque, an encoder 67 to measure the speed of the motor shaft and provide feedback to the control circuitry, and control circuity 68 for receiving control signals and actuating the drive unit. Each drive unit 63 being independent controlled and motorized, the instrument driver 62 may provide multiple (four as shown in FIG. 15) independent drive outputs to the medical instrument. In operation, the control circuitry 68 would receive a control signal, transmit a motor signal to the motor 66, compare the resulting motor speed as measured by the encoder 67 with the desired speed, and modulate the motor signal to generate the desired torque.

For procedures that require a sterile environment, the robotic system may incorporate a drive interface, such as a sterile adapter connected to a sterile drape, that sits between the instrument driver and the medical instrument. The chief purpose of the sterile adapter is to transfer angular motion from the drive shafts of the instrument driver to the drive inputs of the instrument while maintaining physical separation, and thus sterility, between the drive shafts and drive inputs. Accordingly, an example sterile adapter may comprise of a series of rotational inputs and outputs intended to be mated with the drive shafts of the instrument driver and drive inputs on the instrument. Connected to the sterile adapter, the sterile drape, comprised of a thin, flexible material such as transparent or translucent plastic, is designed to cover the capital equipment, such as the instrument driver, robotic arm, and cart (in a cart-based system) or table (in a table-based system). Use of the drape would allow the capital equipment to be positioned proximate to the patient while still being located in an area not requiring sterilization (i.e., non-sterile field). On the other side of the sterile drape, the medical instrument may interface with the patient in an area requiring sterilization (i.e., sterile field).

### D. Medical Instrument.

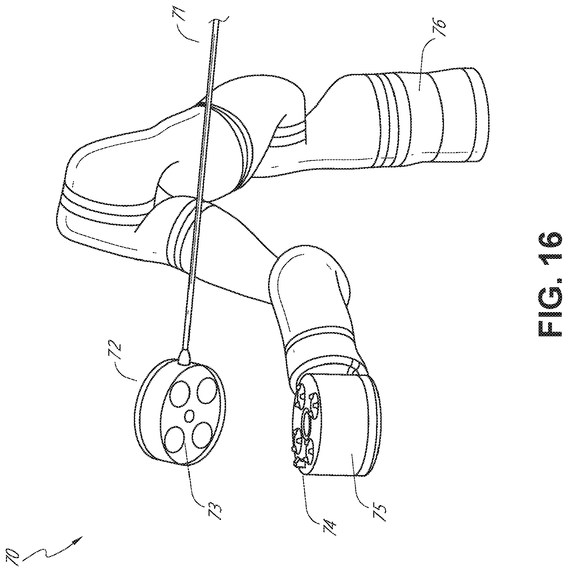

FIG. 16 illustrates an example medical instrument with a paired instrument driver. Like other instruments designed for use with a robotic system, medical instrument 70 comprises an elongated shaft 71 (or elongate body) and an instrument base 72. The instrument base 72, also referred to as an "instrument handle" due to its intended design for manual interaction by the physician, may generally comprise rotatable drive inputs 73, e.g., receptacles, pulleys or spools, that are designed to be mated with drive outputs 74 that extend through a drive interface on instrument driver 75 at the distal end of robotic arm 76. When physically connected, latched, and/or coupled, the mated drive inputs 73 of instrument base 72 may share axes of rotation with the drive outputs 74 in the instrument driver 75 to allow the transfer of torque from drive outputs 74 to drive inputs 73. In some embodiments, the drive outputs 74 may comprise splines that are designed to mate with receptacles on the drive inputs 73.

The elongated shaft 71 is designed to be delivered through either an anatomical opening or lumen, e.g., as in endoscopy, or a minimally invasive incision, e.g., as in laparoscopy. The elongated shaft 71 may be either flexible (e.g., having properties similar to an endoscope) or rigid (e.g., having properties similar to a laparoscope) or contain a customized combination of both flexible and rigid portions. When designed for laparoscopy, the distal end of a rigid elongated shaft may be connected to an end effector extending from a jointed wrist formed from a clevis with at least one degree of freedom and a surgical tool or medical instrument, such as, for example, a grasper or scissors, that may be actuated based on force from the tendons as the drive inputs rotate in response to torque received from the drive outputs 74 of the instrument driver 75. When designed for endoscopy, the distal end of a flexible elongated shaft may include a steerable or controllable bending section that may be articulated and bent based on torque received from the drive outputs 74 of the instrument driver 75.

Torque from the instrument driver 75 is transmitted down the elongated shaft 71 using tendons along the shaft 71. These individual tendons, such as pull wires, may be individually anchored to individual drive inputs 73 within the instrument handle 72. From the handle 72, the tendons are directed down one or more pull lumens along the elongated shaft 71 and anchored at the distal portion of the elongated shaft 71, or in the wrist at the distal portion of the elongated shaft. During a surgical procedure, such as a laparoscopic, endoscopic or hybrid procedure, these tendons may be coupled to a distally mounted end effector, such as a wrist, grasper, or scissor. Under such an arrangement, torque exerted on drive inputs 73 would transfer tension to the tendon, thereby causing the end effector to actuate in some way. In some embodiments, during a surgical procedure, the tendon may cause a joint to rotate about an axis, thereby causing the end effector to move in one direction or another. Alternatively, the tendon may be connected to one or more jaws of a grasper at distal end of the elongated shaft 71, where tension from the tendon cause the grasper to close.

In endoscopy, the tendons may be coupled to a bending or articulating section positioned along the elongated shaft 71 (e.g., at the distal end) via adhesive, control ring, or other mechanical fixation. When fixedly attached to the distal end of a bending section, torque exerted on drive inputs 73 would be transmitted down the tendons, causing the softer, bending section (sometimes referred to as the articulable section or region) to bend or articulate. Along the non-bending sections, it may be advantageous to spiral or helix the individual pull lumens that direct the individual tendons along (or inside) the walls of the endoscope shaft to balance the radial forces that result from tension in the pull wires. The angle of the spiraling and/or spacing there between may be altered or engineered for specific purposes, wherein tighter spiraling exhibits lesser shaft compression under load forces, while lower amounts of spiraling results in greater shaft compression under load forces, but also exhibits limits bending. On the other end of the spectrum, the pull lumens may be directed parallel to the longitudinal axis of the elongated shaft 71 to allow for controlled articulation in the desired bending or articulable sections.

In endoscopy, the elongated shaft 71 houses a number of components to assist with the robotic procedure. The shaft may comprise of a working channel for deploying surgical tools (or medical instruments), irrigation, and/or aspiration to the operative region at the distal end of the shaft 71. The shaft 71 may also accommodate wires and/or optical fibers to transfer signals to/from an optical assembly at the distal tip, which may include of an optical camera. The shaft 71 may also accommodate optical fibers to carry light from proximally-located light sources, such as light emitting diodes, to the distal end of the shaft.

At the distal end of the instrument 70, the distal tip may also comprise the opening of a working channel for delivering tools for diagnostic and/or therapy, irrigation, and aspiration to an operative site. The distal tip may also include a port for a camera, such as a fiberscope or a digital camera, to capture images of an internal anatomical space. Relatedly, the distal tip may also include ports for light sources for illuminating the anatomical space when using the camera.

In the example of FIG. 16, the drive shaft axes, and thus the drive input axes, are orthogonal to the axis of the elongated shaft. This arrangement, however, complicates roll capabilities for the elongated shaft 71. Rolling the elongated shaft 71 along its axis while keeping the drive inputs 73 static results in undesirable tangling of the tendons as they extend off the drive inputs 73 and enter pull lumens within the elongated shaft 71. The resulting entanglement of such tendons may disrupt any control algorithms intended to predict movement of the flexible elongated shaft during an endoscopic procedure.

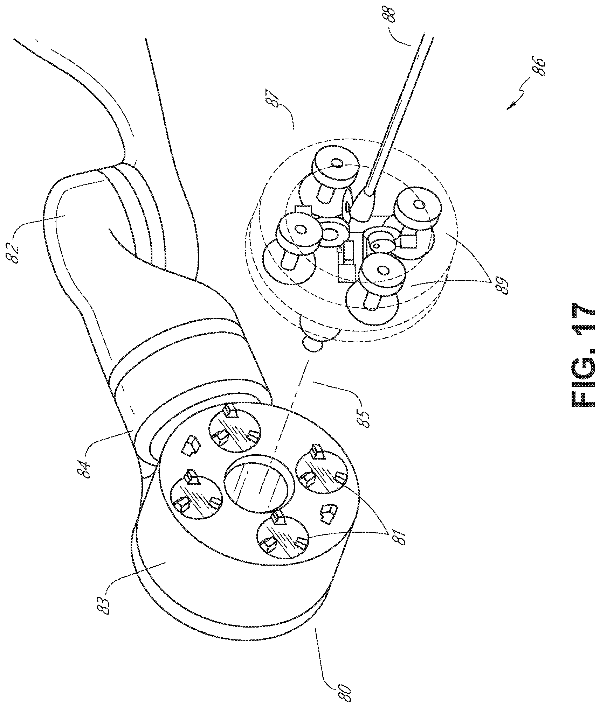

FIG. 17 illustrates an alternative design for an instrument driver and instrument where the axes of the drive units are parallel to the axis of the elongated shaft of the instrument. As shown, a circular instrument driver 80 comprises four drive units with their drive outputs 81 aligned in parallel at the end of a robotic arm 82. The drive units, and their respective drive outputs 81, are housed in a rotational assembly 83 of the instrument driver 80 that is driven by one of the drive units within the assembly 83. In response to torque provided by the rotational drive unit, the rotational assembly 83 rotates along a circular bearing that connects the rotational assembly 83 to the non-rotational portion 84 of the instrument driver. Power and controls signals may be communicated from the non-rotational portion 84 of the instrument driver 80 to the rotational assembly 83 through electrical contacts may be maintained through rotation by a brushed slip ring connection (not shown). In other embodiments, the rotational assembly 83 may be responsive to a separate drive unit that is integrated into the non-rotatable portion 84, and thus not in parallel to the other drive units. The rotational mechanism 83 allows the instrument driver 80 to rotate the drive units, and their respective drive outputs 81, as a single unit around an instrument driver axis 85.

Like earlier disclosed embodiments, an instrument 86 may comprise an elongated shaft portion 88 and an instrument base 87 (shown with a transparent external skin for discussion purposes) comprising a plurality of drive inputs 89 (such as receptacles, pulleys, and spools) that are configured to receive the drive outputs 81 in the instrument driver 80. Unlike prior disclosed embodiments, instrument shaft 88 extends from the center of instrument base 87 with an axis substantially parallel to the axes of the drive inputs 89, rather than orthogonal as in the design of FIG. 16.

When coupled to the rotational assembly 83 of the instrument driver 80, the medical instrument 86, comprising instrument base 87 and instrument shaft 88, rotates in combination with the rotational assembly 83 about the instrument driver axis 85. Since the instrument shaft 88 is positioned at the center of instrument base 87, the instrument shaft 88 is coaxial with instrument driver axis 85 when attached. Thus, rotation of the rotational assembly 83 causes the instrument shaft 88 to rotate about its own longitudinal axis. Moreover, as the instrument base 87 rotates with the instrument shaft 88, any tendons connected to the drive inputs 89 in the instrument base 87 are not tangled during rotation. Accordingly, the parallelism of the axes of the drive outputs 81, drive inputs 89, and instrument shaft 88 allows for the shaft rotation without tangling any control tendons.

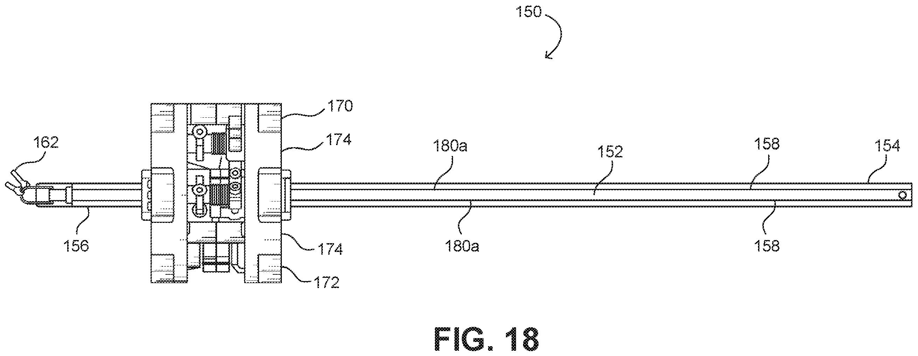

FIG. 18 illustrates an instrument having an instrument based insertion architecture in accordance with some embodiments. The instrument 150 can be coupled to any of the instrument drivers discussed above. The instrument 150 comprises an elongated shaft 152, an end effector 162 connected to the shaft 152, and a handle 170 coupled to the shaft 152. The elongated shaft 152 comprises a tubular member having a proximal portion 154 and a distal portion 156. The elongated shaft 152 comprises one or more channels or grooves 158 along its outer surface. The grooves 158 are configured to receive one or more wires or cables 180 therethrough. One or more cables 180 thus run along an outer surface of the elongated shaft 152. In other embodiments, cables 180 can also run through the elongated shaft 152. Manipulation of the one or more cables 180 (e.g., via an instrument driver) results in actuation of the end effector 162.

The instrument handle 170, which may also be referred to as an instrument base, may generally comprise an attachment interface 172 having one or more mechanical inputs 174, e.g., receptacles, pulleys or spools, that are designed to be reciprocally mated with one or more torque couplers on an attachment surface of an instrument driver.

In some embodiments, the instrument 150 comprises a series of pulleys or cables that enable the elongated shaft 152 to translate relative to the handle 170. In other words, the instrument 150 itself comprises an instrument-based insertion architecture that accommodates insertion of the instrument, thereby minimizing the reliance on a robot arm to provide insertion of the instrument 150. In other embodiments, a robotic arm can be largely responsible for instrument insertion.

### E. Controller.

Any of the robotic systems described herein can include an input device or controller for manipulating an instrument attached to a robotic arm. In some embodiments, the controller can be coupled (e.g., communicatively, electronically, electrically, wirelessly and/or mechanically) with an instrument such that manipulation of the controller causes a corresponding manipulation of the instrument e.g., via master slave control.

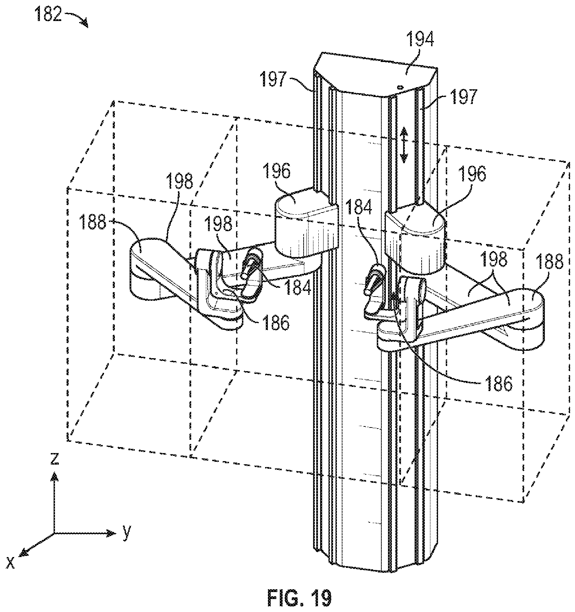

FIG. 19 is a perspective view of an embodiment of a controller 182. In the present embodiment, the controller 182 comprises a hybrid controller that can have both impedance and admittance control. In other embodiments, the controller 182 can utilize just impedance or passive control. In other embodiments, the controller 182 can utilize just admittance control. By being a hybrid controller, the controller 182 advantageously can have a lower perceived inertia while in use.

In the illustrated embodiment, the controller 182 is configured to allow manipulation of two medical instruments, and includes two handles 184. Each of the handles 184 is connected to a gimbal 186. Each gimbal 186 is connected to a positioning platform 188.

As shown in FIG. 19, each positioning platform 188 includes a SCARA arm (selective compliance assembly robot arm) 198 coupled to a column 194 by a prismatic joint 196. The prismatic joints 196 are configured to translate along the column 194 (e.g., along rails 197) to allow each of the handles 184 to be translated in the z-direction, providing a first degree of freedom. The SCARA arm 198 is configured to allow motion of the handle 184 in an x-y plane, providing two additional degrees of freedom.

In some embodiments, one or more load cells are positioned in the controller. For example, in some embodiments, a load cell (not shown) is positioned in the body of each of the gimbals 186. By providing a load cell, portions of the controller 182 are capable of operating under admittance control, thereby advantageously reducing the perceived inertia of the controller while in use. In some embodiments, the positioning platform 188 is configured for admittance control, while the gimbal 186 is configured for impedance control. In other embodiments, the gimbal 186 is configured for admittance control, while the positioning platform 188 is configured for impedance control. Accordingly, for some embodiments, the translational or positional degrees of freedom of the positioning platform 188 can rely on admittance control, while the rotational degrees of freedom of the gimbal 186 rely on impedance control.

### F. Navigation and Control.

Traditional endoscopy may involve the use of fluoroscopy (e.g., as may be delivered through a C-arm) and other forms of radiation-based imaging modalities to provide endoluminal guidance to an operator physician. In contrast, the robotic systems contemplated by this disclosure can provide for non-radiation-based navigational and localization means to reduce physician exposure to radiation and reduce the amount of equipment within the operating room. As used herein, the term "localization" may refer to determining and/or monitoring the position of objects in a reference coordinate system. Technologies such as pre-operative mapping, computer vision, real-time EM tracking, and robot command data may be used individually or in combination to achieve a radiation-free operating environment. In other cases, where radiation-based imaging modalities are still used, the pre-operative mapping, computer vision, real-time EM tracking, and robot command data may be used individually or in combination to improve upon the information obtained solely through radiation-based imaging modalities.

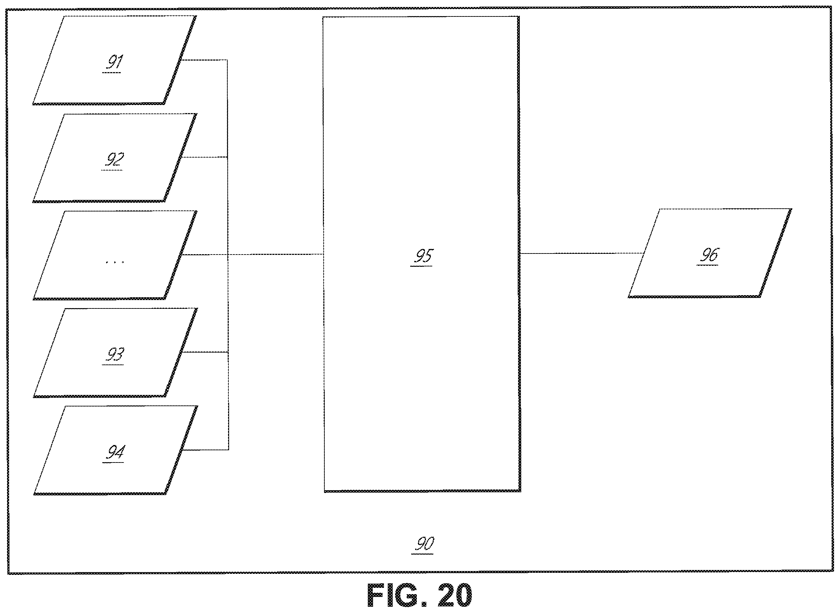

FIG. 20 is a block diagram illustrating a localization system 90 that estimates a location of one or more elements of the robotic system, such as the location of the instrument, in accordance to an example embodiment. The localization system 90 may be a set of one or more computer devices configured to execute one or more instructions. The computer devices may be embodied by a processor (or processors) and computer-readable memory in one or more components discussed above. By way of example and not limitation, the computer devices may be in the tower 30 shown in FIG. 1, the cart shown in FIGS. 1-4, the beds shown in FIGS. 5-14, etc.

As shown in FIG. 20, the localization system 90 may include a localization module 95 that processes input data 91-94 to generate location data 96 for the distal tip of a medical instrument. The location data 96 may be data or logic that represents a location and/or orientation of the distal end of the instrument relative to a frame of reference. The frame of reference can be a frame of reference relative to the anatomy of the patient or to a known object, such as an EM field generator (see discussion below for the EM field generator).

The various input data 91-94 are now described in greater detail. Pre-operative mapping may be accomplished through the use of the collection of low dose CT scans. Pre-operative CT scans are reconstructed into three-dimensional images, which are visualized, e.g. as "slices" of a cutaway view of the patient's internal anatomy. When analyzed in the aggregate, image-based models for anatomical cavities, spaces and structures of the patient's anatomy, such as a patient lung network, may be generated. Techniques such as center-line geometry may be determined and approximated from the CT images to develop a three-dimensional volume of the patient's anatomy, referred to as model data 91 (also referred to as "preoperative model data" when generated using only preoperative CT scans). The use of center-line geometry is discussed in U.S. Pat. App. No. 14/523,760. Network topological models may also be derived from the CT-images, and are particularly appropriate for bronchoscopy.

In some embodiments, the instrument may be equipped with a camera to provide vision data 92. The localization module 95 may process the vision data to enable one or more vision-based location tracking. For example, the preoperative model data may be used in conjunction with the vision data 92 to enable computer vision-based tracking of the medical instrument (e.g., an endoscope or an instrument advance through a working channel of the endoscope). For example, using the preoperative model data 91, the robotic system may generate a library of expected endoscopic images from the model based on the expected path of travel of the endoscope, each image linked to a location within the model. Intra-operatively, this library may be referenced by the robotic system in order to compare real-time images captured at the camera (e.g., a camera at a distal end of the endoscope) to those in the image library to assist localization.

Other computer vision-based tracking techniques use feature tracking to determine motion of the camera, and thus the endoscope. Some features of the localization module 95 may identify circular geometries in the preoperative model data 91 that correspond to anatomical lumens and track the change of those geometries to determine which anatomical lumen was selected, as well as the relative rotational and/or translational motion of the camera. Use of a topological map may further enhance vision-based algorithms or techniques.

Optical flow, another computer vision-based technique, may analyze the displacement and translation of image pixels in a video sequence in the vision data 92 to infer camera movement. Examples of optical flow techniques may include motion detection, object segmentation calculations, luminance, motion compensated encoding, stereo disparity measurement, etc. Through the comparison of multiple frames over multiple iterations, movement and location of the camera (and thus the endoscope) may be determined.

The localization module 95 may use real-time EM tracking to generate a real-time location of the endoscope in a global coordinate system that may be registered to the patient's anatomy, represented by the preoperative model. In EM tracking, an EM sensor (or tracker) comprising of one or more sensor coils embedded in one or more locations and orientations in a medical instrument (e.g., an endoscopic tool) measures the variation in the EM field created by one or more static EM field generators positioned at a known location. The location information detected by the EM sensors is stored as EM data 93. The EM field generator (or transmitter), may be placed close to the patient to create a low intensity magnetic field that the embedded sensor may detect. The magnetic field induces small currents in the sensor coils of the EM sensor, which may be analyzed to determine the distance and angle between the EM sensor and the EM field generator. These distances and orientations may be intra-operatively "registered" to the patient anatomy (e.g., the preoperative model) in order to determine the geometric transformation that aligns a single location in the coordinate system with a position in the pre-operative model of the patient's anatomy. Once registered, an embedded EM tracker in one or more positions of the medical instrument (e.g., the distal tip of an endoscope) may provide real-time indications of the progression of the medical instrument through the patient's anatomy.

Robotic command and kinematics data 94 may also be used by the localization module 95 to provide localization data 96 for the robotic system. Device pitch and yaw resulting from articulation commands may be determined during pre-operative calibration. Intra-operatively, these calibration measurements may be used in combination with known insertion depth information to estimate the position of the instrument. Alternatively, these calculations may be analyzed in combination with EM, vision, and/or topological modeling to estimate the position of the medical instrument within the network.

As FIG. 20 shows, a number of other input data can be used by the localization module 95. For example, although not shown in FIG. 20, an instrument utilizing shape-sensing fiber can provide shape data that the localization module 95 can use to determine the location and shape of the instrument.

The localization module 95 may use the input data 91-94 in combination(s). In some cases, such a combination may use a probabilistic approach where the localization module 95 assigns a confidence weight to the location determined from each of the input data 91-94. Thus, where the EM data may not be reliable (as may be the case where there is EM interference) the confidence of the location determined by the EM data 93 can be decrease and the localization module 95 may rely more heavily on the vision data 92 and/or the robotic command and kinematics data 94.

As discussed above, the robotic systems discussed herein may be designed to incorporate a combination of one or more of the technologies above. The robotic system's computer-based control system, based in the tower, bed and/or cart, may store computer program instructions, for example, within a non-transitory computer-readable storage medium such as a persistent magnetic storage drive, solid state drive, or the like, that, upon execution, cause the system to receive and analyze sensor data and user commands, generate control signals throughout the system, and display the navigational and localization data, such as the position of the instrument within the global coordinate system, anatomical map, etc.

### 2. Cannula.

Embodiments of the present disclosure relate to cannulas that may be utilized to provide access for a surgical procedure. In accordance with embodiments described herein, a cannula may be configured as an elongate device having a passage through which a medical instrument can be introduced into a patient. The cannula may be configured to be installed at an incision or a natural orifice to facilitate safe passage of the medical instrument through the cannula to an internal anatomical site. In some embodiments, the cannula may be configured with a seal to form a trocar that maintains pressure or fluids within the targeted site while allowing the instrument to pass through the cannula.

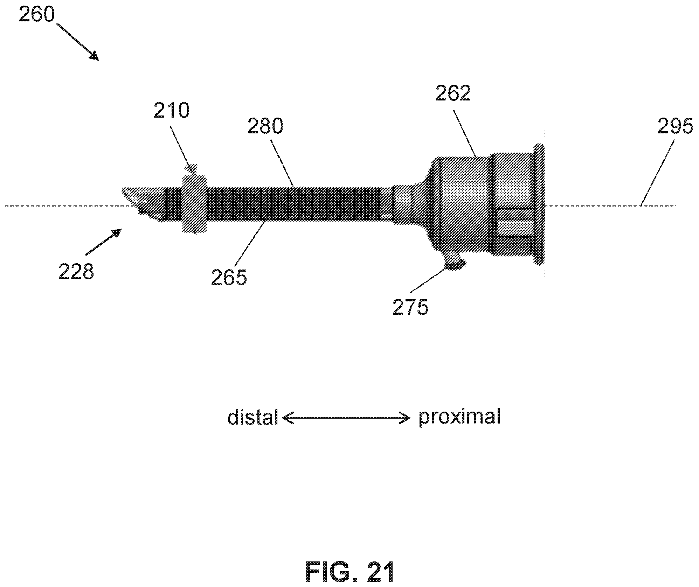

With reference to FIGS. 21, an exemplary cannula 260 is shown. In the depicted example, the cannula 260 may displace or dissect soft tissue to allow the cannula 260 to be inserted into the patient cavity to provide access to a surgical site. Optionally, an obturator may be inserted through the cannula 260 during an initial insertion to provide a dilating or blunt tip that facilitates insertion of the cannula 260 through an incision or other opening on the patient. According to some embodiments, the cannula 260 is configured to provide access to a surgical site in the patient's abdomen for laparoscopic procedures. Additionally or alternatively, the cannula 260 can be configured to provide access to a site for urological, endoscopic, percutaneous, orthopedic, and/or or other medical or minimally invasive procedures in which a medical instrument is introduced to the site through the cannula 260. In some applications, the cannula 260 provides access to a surgical site for robotic procedures performed by robotic systems described herein. Additionally or alternatively, the cannula 260 may be configured for use in manual procedures.

In the depicted example, a proximal portion of the cannula 260 is configured as a funnel portion 262, which has a generally larger diameter than the cannula shaft 280. The funnel portion 262 may transition to the smaller diameter cannula shaft 280 to facilitate insertion of tools from an opening at the proximal end of the cannula 260. Optionally, the funnel portion 262 of the cannula can be utilized by clinicians or other users as a handle to advance the cannula 260 or to otherwise apply force to the cannula 260. In some embodiments, the funnel portion 262 may form an attachment point that can be engaged by a robotic manipulator to manipulate movement of the cannula 260. In some embodiments, the funnel portion 262 includes a removable or fixed seal that aids in sealing fluids, such as insufflation gas or liquids, within a patient cavity. A fluid port 275 (such as a stop cock or luer fitting) may be positioned at the funnel portion 262 of the cannula 260 to provide connection point through which fluids may flow through the cannula 260 and into or out of the patient.

As illustrated, cannula shaft 280 is configured as a tubular portion that extends distally from the funnel portion 262. A lumen of the cannula shaft 280 provides a passage through which a shaft of an instrument may extend to access a surgical site. The cannula lumen can provide a working corridor or working channel through which tools can be inserted, manipulated, and/or removed along a longitudinal axis 295 of the cannula. Optionally, the movement of the cannula 260 and the tools can be manipulated or controlled by robotic systems described herein. In the illustrated embodiment, the cannula shaft 280 is configured as a straight tube that terminates at a beveled distal end 228, but the cannula 260 and cannula shaft 280 may have a variety of configurations. For example, the cannula shaft 280 may be curved, bent, or angled, or the distal end of the cannula shaft 280 may be flat or have other geometries.

As seen in FIG. 21, cannula 260 may include a series of ridges 265 arranged along an outer surface of the cannula shaft 280. The ridges 265 may be configured to engage a tissue wall of the patient when the cannula is inserted into the patient, aiding in placement or stability of the cannula 260. The ridges may include a series of peaks and valleys that are arranged axially along all or a portion of the cannula shaft 280. As the cannula 260 is advanced distally or retracted proximally, the ridges may slide against the patient tissue wall and may provide tactile feedback to the clinician. Additionally or alternatively, the ridges 265 may serve to retain the cannula 260 at a desired insertion depth and stabilize the cannula 260 within the patient.

As seen in FIG. 21, the cannula 260 may include a defined remote center of motion 210 at a location along the cannula shaft 280. The remote center of motion 210 (also referred to herein as simply a "remote center" or "RCM") may correspond to a predetermined axial location along the cannula shaft 280 about which the cannula is pivoted by a robotic system when movement of the cannula is manipulated robotically. The remote center 210 may also indicate a desired insertion depth of the cannula 260, where the cannula shaft 280 is designed to intersect with a body wall layer of the patient.

### 3. Docking.

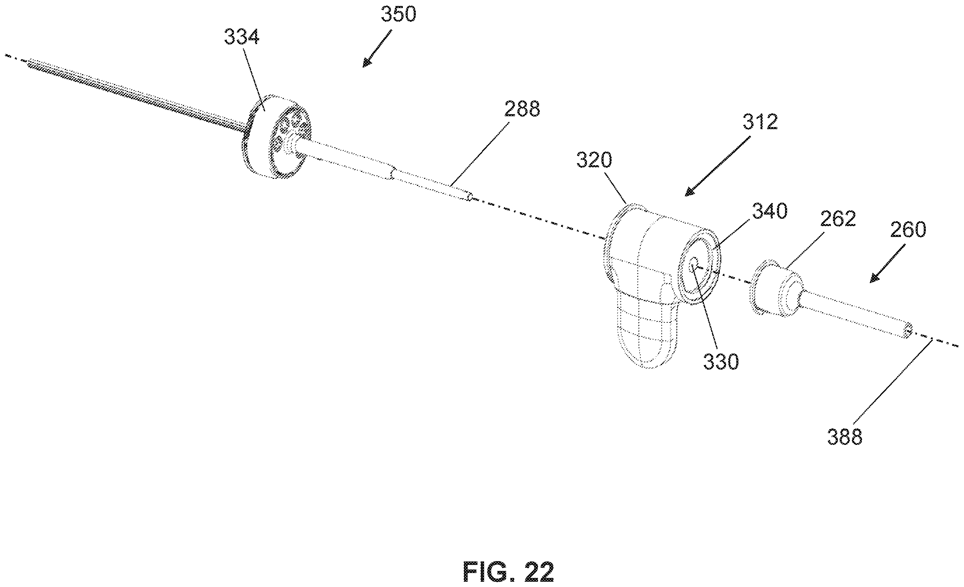

FIG. 22 depicts an exemplary system where a manipulator 312 of a robotic system is configured to manipulate cannula 260 and a medical instrument 350 inserted through the cannula 260. The manipulator 312 may be coupled to a distal end of a robotic arm and may be configured in accordance with any of the manipulators or instrument drivers described herein (e.g., 28, 62, 75, 80). The medical instrument 350 may include an elongate instrument shaft 288 and may be configured in accordance with any of the medical instruments described herein (e.g., 34, 70, 88, 150).

Manipulator 312 may be configured to dock to medical devices, such as the cannula 260 and/or the medical instrument 350, such that the manipulator may control movement of these devices. FIG. 22 illustrates various components of the system in an undocked configuration for purposes of illustration. As seen in FIG. 22, manipulator 312 includes an instrument dock 320, configured to attach to a base 334 of the medical instrument 350, and a cannula dock 340, configured to attach to the funnel portion 262 of the cannula 260. In the illustrated embodiment, the instrument dock 320 is positioned on a first side of the manipulator 312 and the cannula dock 340 is positioned on a second side of the manipulator 312. The manipulator 312 further includes a passage 330 to accommodate the shaft 288 of the medical instrument 350 and permit the shaft 288 of the medical instrument 350 to extend through the manipulator 312.

Each of the docks 320, 340 may include a suitable attachment mechanism (e.g., a latch) that facilitates docking and undocking of the respective device while securing the device to a body of the manipulator 312 when the device is in a docked configuration. In use, the cannula 260 may be inserted into a patient at a desired entry opening for the procedure. The manipulator 312 may then be moved to the position of the cannula 260, and the cannula dock 340 may be attached to the cannula 260. The instrument 350 may then be inserted through the cannula 260 and attached to the instrument dock 320. When in a docked configuration, the instrument 350 and cannula 260 may be coaxially aligned along an insertion axis 388 such that the shaft 288 can extend through the passage 330 and the cannula 260.

While FIG. 22 depicts a single manipulator docked to both the instrument 350 and the cannula 260, it is possible for multiple robotic manipulators or arms to be employed such that movement of the cannula 260 and the instrument 350 are controlled by separate robotic manipulator or arms. Further, the manipulator 312 may be configured to attach to the instrument 350 and the cannula 260 directly, or the manipulator 312 may be configured to attach to the instrument 350 and the cannula 260 through an intervening sterile barrier.

### 4. Remote Center Movement.

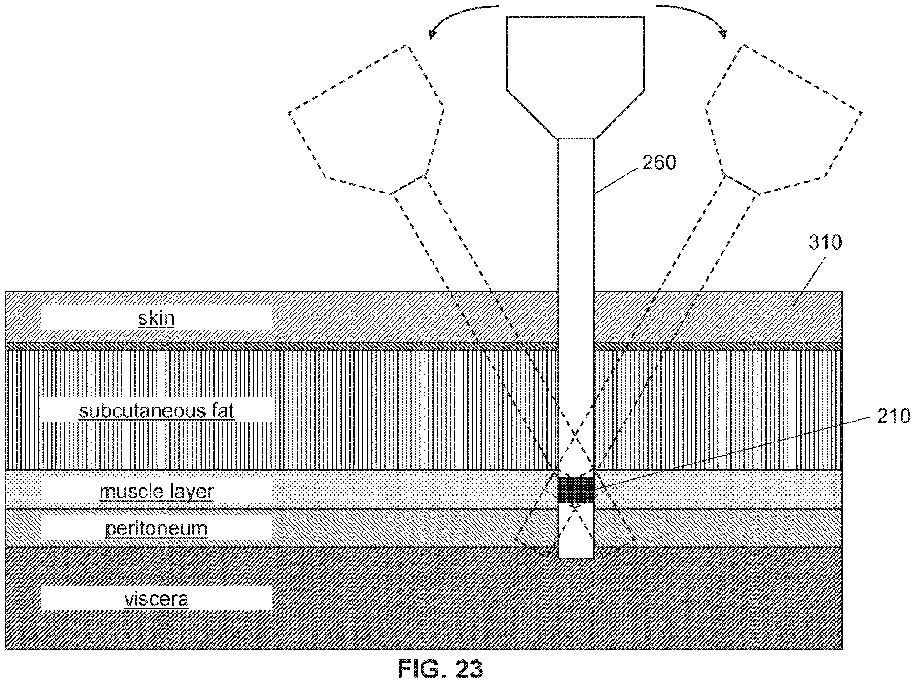

FIG. 23 illustrates a front view of a cannula 260 inserted into a patient's tissue 310 to access the patient cavity. During operation, the cannula 260 can be pivoted to move the cannula 260 within the patient cavity to provide access to various areas within the patient cavity. In some applications, the pivot point, or remote center of motion 210 of the cannula 260 can be established by a robotic surgery system.

As can be appreciated, the robotic arm and/or surgical system can establish and maintain the position of the remote center of motion 210 for the instrument and/or for a cannula 260. Depending on the implementation, the remote center of motion 210 can be maintained either mechanically or by software executed on one or more processors of the system. During a surgical procedure, the instrument may be inserted through the patient's body wall 310 to gain access to an internal region of the patient, via a cannula 260. The remote center of motion RCM can be located at the intersection between the body wall or patient tissue 310 and the cannula 260, and more particularly at, for example, an axial location along the cannula shaft predetermined to intersect a muscle layer (e.g., of the abdomen) of the body wall where excessive lateral stresses may be undesirable, in order to prevent and/or reduce movement of the body wall during the procedure, thereby enabling the surgical procedure to safely take place. For example, if the location of the intersection between the cannula 260 is not held substantially stationary during the pivoting motions of the cannula 260, the cannula may apply unnecessary force to the body wall, potentially tearing the body wall. Thus, it is desirable to maintain the remote center of motion 210 to prevent unnecessary forces from being applied to the body wall or patient tissue 310. Prior to operation, a clinician can advance the cannula 260 through the patient's tissue 310 such that the remote center of motion 210 is placed at a desired insertion depth, where the remote center intersects a desired layer of the patient's body wall and passes through the patient's tissue to minimize trauma as the cannula is pivoted about the remote center of motion 210.

In some applications, a clinician may not have an indication of where the desired remote center of motion RCM of the cannula 260 is located during insertion or advancement of the cannula 260.

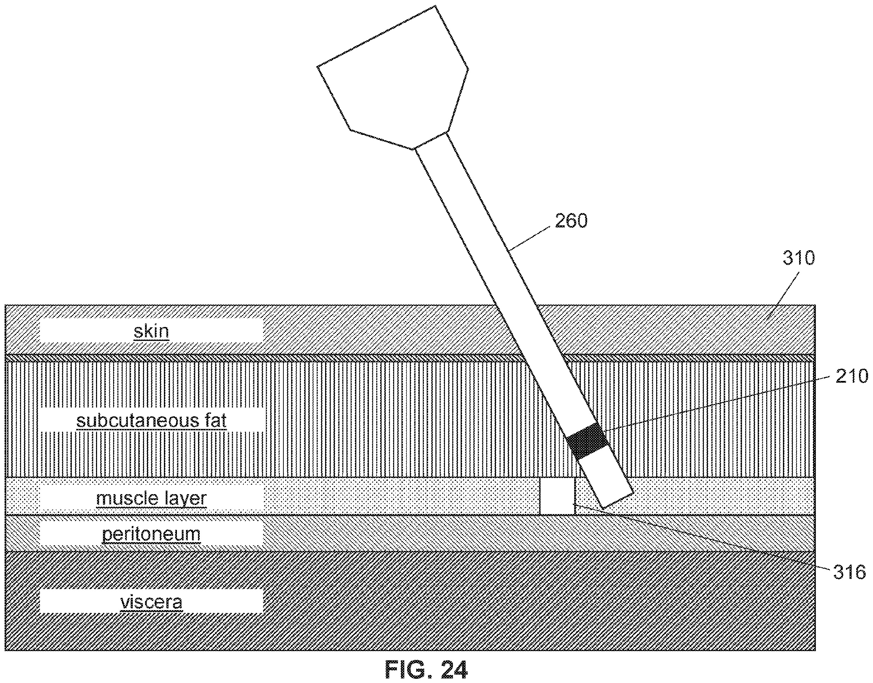

FIG. 24 illustrates a front view of the cannula 260 moved out of alignment with the patient's tissue 310. In some applications, the cannula 260 can move or slip relative to the patient tissue 310, causing the remote center of motion to be moved out of alignment with the patient tissue 310. In some embodiments, such movement of the cannula 260 can cause the cannula to be dislodged from the passage 316 formed through the patient's tissue 310, requiring the cannula 260 to be reinserted through the patient's tissue 310, requiring additional time and potentially causing additional trauma to the patient. In some applications, the cannula 260 can move relative to the patient tissue 310 due to movement of the robotic arm, inadvertent contact, or the positioning of the cannula 260. As can be appreciated, laterally positioned cannulas 260 may be more likely to move relative to the patient tissue 310.

With certain cannulas a clinician may not have an indication of where the desired remote center of motion is located. Further, with certain cannulas, the cannula may slip relative to the patient's tissue, displacing the cannula relative to the patient cavity. Accordingly, during some procedures, the remote center of motion may not be aligned with the patient's tissue, causing trauma as the cannula is moved. Further, in certain applications, the cannula may be inadvertently moved from the patient cavity, requiring reinsertion of the cannula, adding time and risking trauma to the patient.

Advantageously, embodiments disclosed herein can allow a clinician to identify the location of a desired remote center of motion. Further, in some embodiments, the cannulas can include features that facilitate retention of the cannulas in a desired position relative to the patient cavity, preventing the cannula from being displaced and allowing more control when adjusting the position of the cannula within the patient cavity. As can be appreciated, the configuration of the disclosed cannulas can reduce clinical errors, reduce procedure time, and simplify workflow.

FIG. 25 illustrates a front view of a cannula 460. The cannula 460 can be configured in accordance with any of the cannulas described herein and can include any of the features described with respect to cannulas herein (e.g., cannula 260). In the depicted example, the cannula 460 provides access to the patient cavity. In some applications, the cannula 460 provides access to a surgical site for robotic laparoscopic procedures performed by robotic systems described herein. Optionally, the movement of the cannula 460 and the tools can be manipulated or controlled by robotic systems described herein. In some embodiments, portions of the cannula 460 can be formed from plastic, metal, or other materials.

As illustrated, the cannula 460 includes a cannula shaft 480 extending from a proximal portion or funnel 462 of the cannula 460. The shaft 480 defines a shaft lumen 482 therein that provides access between the end portion 484 and the funnel 462. As described herein, the end portion 484 of the shaft 480 can be advanced into the patient cavity to allow access to the patient cavity via the funnel 462. Optionally, the end portion 484 can include a beveled portion to displace tissue as the cannula 460 (or the trocar assembly generally) is advanced through the patient cavity. In some applications, an obturator can utilized to assist in advancing the cannula through the patient cavity. The obturator can extend through the cannula lumen.

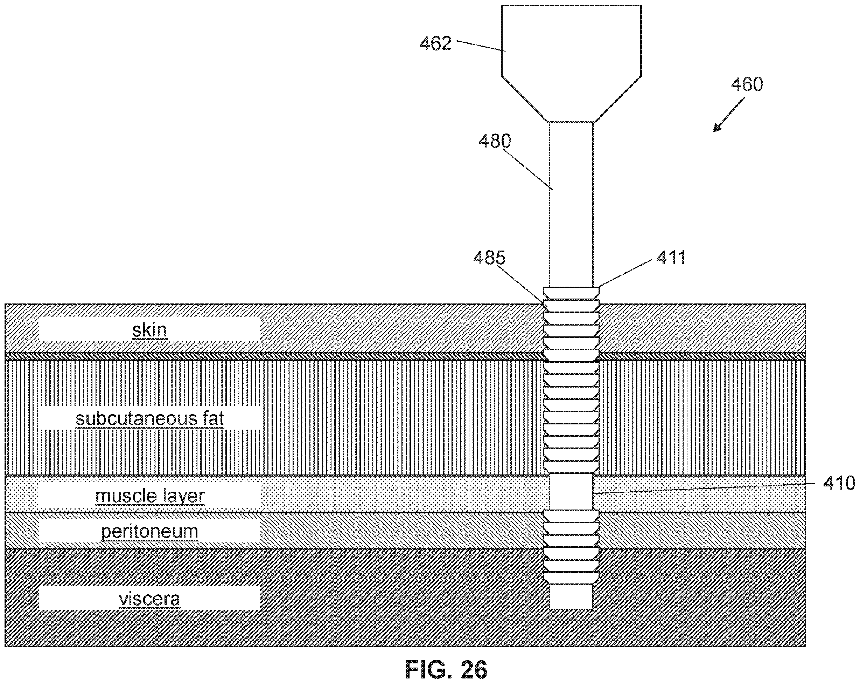

FIG. 26 is a cross-sectional view of the cannula 460 of FIG. 25 inserted into a patient's tissue to access the patient cavity. With reference to FIGS. 25 and 26, the cannula 460 can include features to aid in the placement and stability of the cannula 460 with respect to the patient tissue 310 and with respect to the remote center of motion. In the depicted example, the cannula shaft 480 can include one or more ridges 483 disposed along the outer surface 481. The ridges 483 can be configured to engage or grab onto the peritoneum, thereby preventing or reducing a tendency of the cannula 460 to inadvertently insert or retract during use.

The ridges 483 can be circumferentially formed in the outer surface 481 of the shaft 480. The ridges 483 can form recessed portions along the outer surface 481 of the shaft 480. The ridges 483 can be generally circular around the outer diameter of the cannula shaft to permit the shaft 480 to rotate relative to the patient cavity and the peritoneum 317 of the patient. In some applications, the ridges 483 can include smooth portions and/or textured portions. Optionally, the ridges 483 can be laser etched.

As can be appreciated, the geometry of the ridges 483 can affect the engagement of the ridges 483 with the peritoneum. The ridges 483 can be designed to permit cannula 460 insertion and removal with minimal patient trauma while providing intra-operative stability, allowing the cannulas 460 to remain in position during use. In some embodiments, the ridges 483 can include tapered edges to adjust the insertion and removal resistance of the cannula 460. For example, the ridges 483 can allow for a cannula 460 to have a high removal resistance and a lower insertion resistance. For example, a ridge 483 can have a proximal edge 483b that has a straight or squared edge to increase the removal force of the cannula 460. Further, a ridge 483 can have a distal edge 483a that is tapered to minimize the insertion force as the cannula 460 is advanced through the peritoneum 317. In some embodiments, the radius or slope of the distal edge 483a of a ridge 483 is less than the radius or slope of the proximal edge 483b of the ridge 483.

As illustrated, the cannula 460 can include multiple ridges 483 disposed along the outer surface 481. Optionally, the ridges 483 can have varying depths and lengths. As illustrated, multiple ridges 483 can be grouped together along the outer surface 481 in segments 485. Segments 485 of ridges are separated by a non-ridged segment 486 of the cannula shaft.

In some embodiments, the ridges 483 are disposed along the length of the cannula shaft 480 in groups or segments 485 of varying quantities. Optionally, the ridges 483 can be disposed along a portion or portions of the shaft 480. The various portions or segments 485 of the ridges 483 can be spaced apart. The pitch or spacing between the ridges 483 and/or the segments 485 can be even or varied. Each segment 485 of ridges 483 can include ridges 483 of the same or similar geometry or ridges 483 of differing geometry. As described herein, the spacing and geometry of the ridges 483 can indicate particular portions of the cannula 460, including, but not limited to, various remote center of motions identified for the cannula 460.

In the depicted example, the cannula 460 allows for the remote center of motion to be identified by a clinician. In some embodiments, the ridges 483 described herein can be utilized as an identifier of the location of the remote center of motion RCM. In some embodiments, the ridges 483 or segments 485 of ridges can define a pattern to identify the location of the remote center of motion. In some embodiments, the geometry of the ridges 483 provides tactile feedback as the cannula 460 is positioned to align a remote center of mass RCM with the peritoneum 317 of the patient.

In FIG. 25, non-ridged segment 486 is co-located with and axially overlaps with remote center 410, such that the non-ridged segment 486 provides a visual and tactile indication of the location of the remote center 410. Further, because ridged segments 485 are positioned proximal and distal to the location of the remote center 410, the ridges 483 engage the tissue wall to facilitate retention of the cannula 460 with the remote center 410 at the desired insertion depth.

In some embodiments, the pattern of ridges may provide an indication of proximity to the remote center. For example, as seen in FIG. 26, a segment 485 of ridges can be spaced apart from the defined remote center of motion 410 by a fixed distance, such that when the defined remote center of motion is at the proper insertion depth within the patient, the proximal (upper) end 411 of the segment 485 is visible outside of the patient, confirming proper insertion of the cannula 460. Further, if the cannula 460 is inserted deeper than the proper insertion depth, such that the remote center of motion is misaligned, the proximal end of the segment 485 can be obscured within the patient.

As illustrated, an indicator or identifier 487 can be defined by the absence of ridges 483 or spacing between ridges 483 to define the location of a remote center of motion RCM. Optionally, the indicator can include a smooth segment between ridges 483 and/or a textured segment between ridges 483. The textured segment of the indicator can be a different texture than the texture applied to the ridges 483. Optionally, the axial length of the indicator portion is shorter than the axial length of one or more of the ridge segments 485.

In some embodiments, the identifier 487 can include an optical or colored marking. Such markings may, for example, be formed by laser etched portions of the cannula shaft. Therefore, as the cannula 460 is positioned, the indicator can provide visual feedback to align the remote center of motion with the pivot point within the patient. Optionally, the indicator can axially overlap with ridges 483.

Certain robotic systems can utilize multiple robotic arms (e.g., three arms on each side of a patient) to perform very complex medical procedures. The robotic arms can be designed to be optimized for a variety of different poses depending on the type of medical procedure to be performed. These poses can impose different optimization constraints on one or more of the robotic arms' remote center distances. Example optimization constraints may include variables such as the spacing between ports/cannulas, the extension of a robotic arm to reach a port/cannula, collision avoidance, pose optimization (e.g., for singularity avoidance, to improve natural frequency, to maximize workspace, etc.), and instrument reach. In order to accommodate these optimization constraints, the system can enable one or more of the robotic arms to have a variable remote center distance, even within a single treatment episode. Therefore, some embodiments are configurable to allow the cannula to pivot about multiple remote centers of motion, allowing a clinician to select a desired remote center of motion for the cannula. Advantageously, different remote centers of motion can be selected to adjust the access or reach of the cannula within the patient cavity. The use of software-based setting of different remote center distances can enable the system to, for example, move a robotic arm in a null-space DoF and/or increase a maximum distance the medical tool is able to be inserted into the patient.

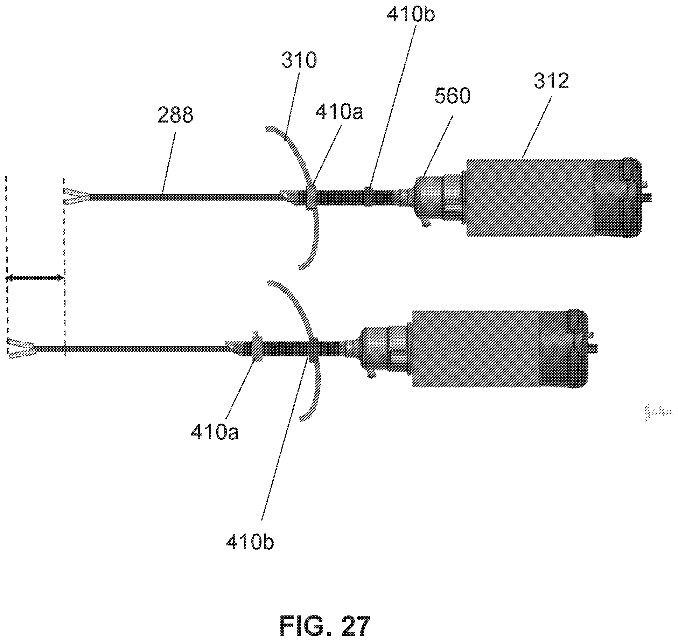

FIG. 27 depicts an example where cannula 560 is configured with multiple remote center locations. Cannula 560 may otherwise be configured in accordance with any of the cannulas described herein. Here, cannula 560 includes a dual remote center configuration including a first defined remote center 410a and a second defined remote center 410b. The use of two discrete remote centers are predetermined axial locations can improve usability by reducing confusion during use, but in other arrangements, more than two remote centers may be defined along the cannula shaft. As seen in FIG. 27, the system may be operated (e.g., intraoperatively) to switch between the first remote center 410a to the second remote center 410b to, for example, extend reach of the instrument 350.

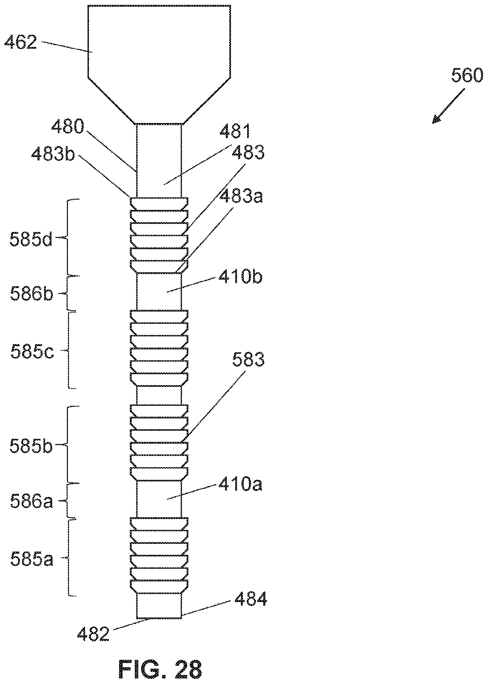

FIG. 28 is a front view of cannula 560. The cannula 560 allows for each remote center of motion 410a, 410b to be identified by a clinician, permitting the clinician to confirm the desired remote center of motion is aligned within the patient.

In some embodiments, the ridges 583 described herein can be utilized as an identifier of the location of each remote center of motion. As can be appreciated, the cannula 560 can include or identify more than two remote centers of motion. In some embodiments, the ridges 583 or segments 585 of ridges can define a pattern to identify the location of each remote center of motion 410a, 410b. As can be appreciated, the upper segments 585d, 585c axially surrounding the upper remote center of motion 410b can define a similar pattern or a different pattern than the lower segments 585a, 585b surrounding the lower remote center of motion 410a. In some embodiments, the geometry of the ridges 583 provides tactile feedback as the cannula 560 is positioned to align a remote center of motion 410a, 410b with the peritoneum of the patient. The ridges 583 of each of the segments 585a, 585b, 585c, and 585d can include different depths, axial lengths, or other geometric features, or the ridges may be uniform in depth, length, geometry, etc.. Each of the segments 585a, 585b, 585c, and 585d can include different numbers of ridges 583 and/or ridge pitches, or the numbers and/or pitches of ridges may be the same. In some embodiments, the ridges 583 can provide different tactile feedback for the proximal (upper) remote center of motion 410b and the distal (lower) remote center of motion 410a,

Optionally, the upper segments 585c, 585d can be spaced apart from the upper remote center of motion 410b by a fixed distance, such that when the defined remote center of motion 410b is at the proper insertion depth within the patient, the upper end of segment 585a is visible outside of the patient, confirming proper insertion of the cannula 560 at the upper remote center of motion 410b. Similarly, the lower segments 585a, 585b can be spaced apart from the lower remote center of motion 410a by a fixed distance, such that when the defined remote center of motion 410a is at the proper insertion depth within the patient, the upper end of segment 585b is visible outside of the patient, confirming proper insertion of the cannula 560 at the lower remote center of motion 410a. Further, if the cannula 560 is inserted deeper than the proper insertion depth for a respective remote center of motion, such that either remote center of motion 410a, 410b is misaligned, the upper end of the segment 585b, 585d can be obscured within the patient.

As illustrated, identifiers can be defined by the absence of ridges 583 at non-ridged segments 586a, 586b interposed between the lower segments 585a,585b and the upper segments 585c, 585d, respectively, indicating the axial location of remote centers of motion 410a, 410b. Optionally, the indicators can include a smooth segment or a textured segment between ridges of the lower segments 585a,585b and the upper segments 585c, 585d. The textured segment of the indicators can be a different texture than the texture applied to the ridges 583. Optionally, the axial length of the indicator portion is shorter than the axial length of one or more of the ridge segments 585a, 585b, 585c, 585d.

In some embodiments, the cannula includes markings to indicate locations of multiple remote centers of motions. The markings may provide visual indicators (e.g., colored or shaded markings) that provide a visible indication of the location of multiple remote centers. In some embodiments, the markings may be distinguishable from each other such that the multiple remote centers may be discriminated from one another. Additionally or alternatively, the markings may indicate a priority for one of the remote centers of the other. As with the non-ridged indicators described above, the markings may be formed as circumferential indicators around the outer surface of the cannula shaft such that the indicators can be observed when the cannula is rolled about its longitudinal axis. In some embodiments, proximity markings are included proximal (above) or distal (below) the remote center indicators to indicate a proximity to the remote center location when the remote center indicator is obscured from view (for example, within the patient body wall). In some embodiments, the proximity markings may include directionality indications such that neighboring proximity marking scan be distinguished from one another and such that the correct remote center corresponding to the proximity marking can be identified.

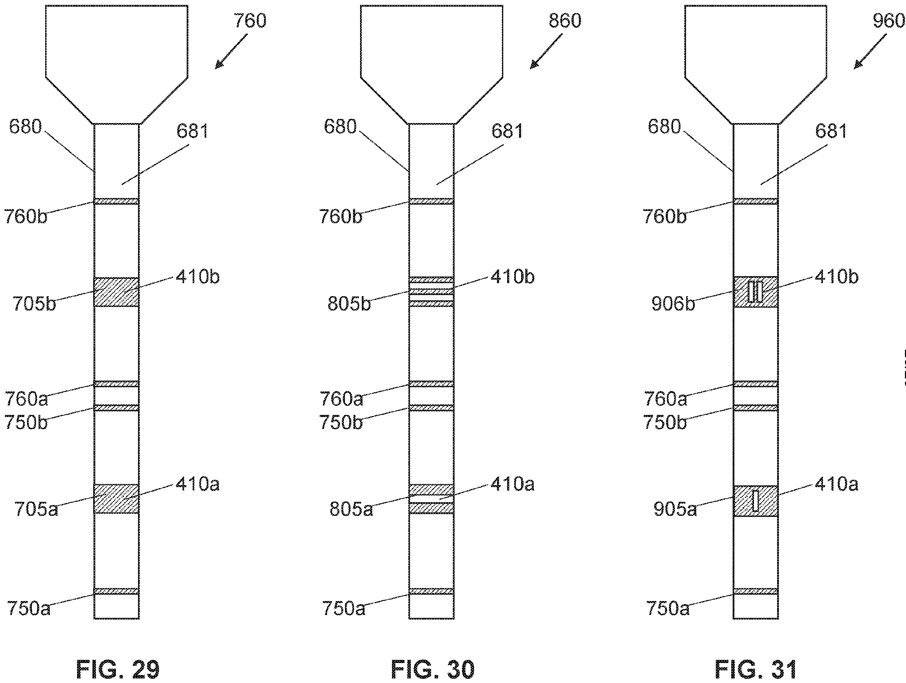

FIGS. 29-33 depict cannulas having multiple sets of markings indicative of multiple defined remote centers 410a, 410b, in accordance with various embodiments. The markings may be laser etched, cut into the outer surface, painted on, or otherwise formed on the surface of the cannula such that the markings are visible to a user. In the illustrated embodiments, the cannula shafts 680 are shown with smooth and non-ridged outer surfaces 681, but in other embodiments, the markings may be integrated with ridges sections of the cannula such as those described above.

With reference to FIG. 29, the cannula 760 includes a first remote center indicator 705a indicative of a first defined remote center 410a, and a second remote center indicator 705b indicative of a second defined remote center 410b. Each of the first and second remote center indicators is formed of a circumferential shaded marking that forms a visible indicator at the axial location corresponding to the respective remote center.

The cannula 760 further includes a first pair of proximity indicators 750a, 750b corresponding to the first remote center 410a, and a second pair of proximity indicators 760a, 760b corresponding to the second remote center 410b. Each proximity indicator is positioned a fixed and predetermined distance axially away from the corresponding remote center along the cannula shaft, such that the proximity indicator indicates proximity to corresponding remote center, and such that the proximity indicator can indicate a location of the corresponding remote center when the remote center is not otherwise visible. As seen in FIG. 29, the first pair of proximity indicators 750a, 750b includes a first distal indicator 750a positioned distal to the first remote center 410a and a first proximal indicator 750b positioned proximal to the first remote center 410a. Similarly, the second pair of proximity indicators 760a, 760b includes a second distal indicator 760a positioned distal to the second remote center 410b (and proximal to the first remote center 410a), and a second proximal indicator 760b positioned proximal to the second remote center 410b. Each of the proximity indicators is also formed of a thinner circumferential band than the remote center indicators such that the proximity indicator markings can be discriminated from the remote center indicator markings.

With reference to FIG. 30, cannula 860 may be similar to cannula 760 or any other cannula herein, but here, the first and second remote center indicators 805a and 805b are made of visually distinct markings such that the first remote center 410a or the second remote center 410b can be identified and discriminated from each other based on their appearance. In this example, each remote center indicator includes a series of horizontal (circumferential markings), wherein the first and second remote centers 805a, 805b have different numbers and/or thicknesses of bands to discriminate one from the other.

With reference to FIG. 31, cannula 960 may be similar to cannula 860 or any other cannula herein, but here, the first and second remote center indicators 905a and 905b are made of vertical bands superimposed on circumferential bands, where different numbers or sizes of the vertical bands are used to discriminate one remote center indicator from the other.

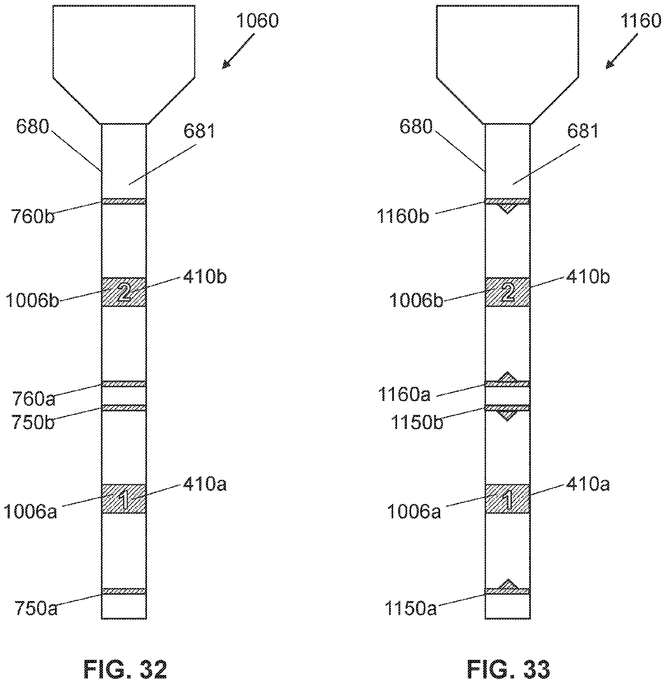

With reference to FIG. 32, cannula 1060 may be similar to cannula 960 or any other cannula herein, but here, the first and second remote center indicators 1005a and 1005b include alphanumeric characters on circumferential bands, where different characters are used to discriminate one remote center indicator from the other. Here, the first remote center 410a is indicated with a first numeral ("1"), and the second remote center 410b is indicated with a second numeral ("2"), but other characters such as other numerals or letters may be used. In this example, the indicators also indicate a priority of the indicators. The first indicator 1006a indicates a primary remote center, which is located more distal along the shaft 680, closer to the tip of the cannula, and which is intended to be used as the default remote center. The second indicator 1006b indicates a secondary remote center, which is located more proximal along the shaft and is intended to be used in special circumstances, such as when additional reach with an instrument is desired, as shown in FIG. 27. The discriminations and indicators of priority may aid the user in understanding which remote center to use during placement or when switching between remote centers.

With reference to FIG. 33, cannula 1160 may be similar to cannula 1060 or any other cannula herein, but here, each of the proximity indicators 1150a,b, 1160a,b further includes a directionality marking pointing in the axial direction of corresponding nearest remote center. Here, the directionality marking is configured as an arrow, but the directionality marking may be configured as any other marking depicting a preferential axial direction. For example, the directionality marking may include a set of one or more graded markings that gradually decreases or otherwise changes in density, size, or color, as the graded markings get closer to the remote center. The directionality can aid a user in understanding a direction of the remote center for the given proximity marking and discriminating between adjacent proximity markings (e.g., 1150b, 1160a).

Although embodiments are described herein with respect to cannulas, remote center and stability systems described herein may be applied to other medical devices. For example, any of the indicators or stability ridges disclosed herein may be applied to an elongate shaft of other medical devices besides cannulas, including, for example, medical instrument shafts and other access devices.

### 3. Implementing Systems and Terminology.

It should be noted that the terms "couple," "coupling," "coupled" or other variations of the word couple as used herein may indicate either an indirect connection or a direct connection. For example, if a first component is "coupled" to a second component, the first component may be either indirectly connected to the second component via another component or directly connected to the second component.

The methods disclosed herein comprise one or more steps or actions for achieving the described method. The method steps and/or actions do not form part of the claimed invention.

As used herein, the term "plurality" denotes two or more. For example, a plurality of components indicates two or more components. The term "determining" encompasses a wide variety of actions and, therefore, "determining" can include calculating, computing, processing, deriving, investigating, looking up (e.g., looking up in a table, a database or another data structure), ascertaining and the like. Also, "determining" can include receiving (e.g., receiving information), accessing (e.g., accessing data in a memory) and the like. Also, "determining" can include resolving, selecting, choosing, establishing and the like.

The phrase "based on" does not mean "based only on," unless expressly specified otherwise. In other words, the phrase "based on" describes both "based only on" and "based at least on."

The previous description of the disclosed implementations is provided to enable any person skilled in the art to make or use the present invention. Various modifications to these implementations will be readily apparent to those skilled in the art, and the generic principles defined herein may be applied to other implementations without departing from the scope of the invention. For example, it will be appreciated that one of ordinary skill in the art will be able to employ a number corresponding alternative and equivalent structural details, such as equivalent ways of fastening, mounting, coupling, or engaging tool components, equivalent mechanisms for producing particular actuation motions, and equivalent mechanisms for delivering electrical energy. Thus, the present invention is not intended to be limited to the implementations shown herein but is defined by the independent claim.

## Claims

1. A robotic medical system comprising:
a cannula (460) having a first defined remote center of motion (410a) and a second defined remote center of motion (410b) spaced apart axially from the first defined remote center, wherein an outer surface (481) of the cannula comprises stability ridges (483) configured to engage a tissue wall of a patient;
an elongate medical instrument (350) configured to be inserted into the patient through the cannula;
a robotic arm (76, 82) configured to manipulate the elongate medical instrument to perform a medical procedure; and
a controller (182) configured to control movement of the robotic arm while maintaining movement about the first defined remote center and to switch from maintaining movement about the first defined remote center to maintaining movement about the second defined remote center,
wherein a first segment (485) of the stability ridges is disposed on a first side of the first defined remote center and a second segment (485) of the stability ridges is disposed on a second side of the first defined remote center axially spaced apart from the first side of the first defined remote center, wherein the first segment of the stability ridges is separated from the second segment of the stability ridges by a non-ridged segment (486) of the cannula, wherein the first defined remote center is positioned at the non-ridged segment, and wherein a third segment (585c) of the stability ridges is disposed on a first side of the second defined remote center and a fourth segment (585d) of the stability ridges is disposed on a second side of the second defined remote center axially spaced apart from the first side of the second defined remote center.

2. The robotic medical system of Claim 1, wherein the cannula further comprises a marking at the first defined remote center.

3. The robotic medical system of Claim 1, wherein:
the robotic arm comprises an instrument manipulator (312) configured to attach to the elongate medical instrument and to the cannula; and
the controller is configured to control movement of the robotic arm so that each of the elongate medical instrument and the cannula pivot about the first defined remote center.

4. The robotic medical system of Claim 1, wherein two or more of the stability ridges are configured to provide different tactile feedback from each other when engaged with the tissue wall of the patient.

5. The robotic medical system of Claim 1, wherein two or more sets of the stability ridges differ from each other in at least one of shape, size, or pitch.

6. The robotic medical system of Claim 1, wherein at least one of the ridges is configured to resist movement against the tissue wall in a first axial direction greater than the ridge resists movement against the tissue wall in a second axial direction opposite the first axial direction.

7. The robotic medical system of Claim 1, wherein an upper end of the first segment of the stability ridges is spaced apart axially from the first defined remote center by a fixed distance such that, when the first defined remote center is at a proper insertion depth in the patient, the upper end is visible from outside the patient, and, when the first defined remote center is deeper than the proper insertion depth, the upper end is obscured within the patient.

8. The robotic medical system of Claim 1, wherein:
the second and third segments of stability ridges are separated by a non-ridged portion (586b) of the cannula; and
an upper end of the third segment of the stability ridges is spaced apart axially from the second defined remote center by a fixed distance such that, when the second defined remote center is at a proper insertion depth in the patient, the upper end of the third segment is visible from outside the patient, and, when the second defined remote center is deeper than the proper insertion depth, the upper end of the third segment is obscured within the patient.

9. The robotic medical system of Claim 1, wherein the cannula further comprises:
a funnel portion (462);
a tubular portion (480) extending distally from the funnel portion;
a visual indicator (487) on the tubular portion indicative of the first defined remote center of motion; and
an inner lumen (482) extending through the tubular portion,
wherein the first segment of ridges is disposed proximal to the visual indicator, and
wherein the second segment of ridges is disposed distal to the visual indicator.

10. The robotic medical system of Claim 9, wherein the ridges comprise at least one tapered ridge having a distal radius and a proximal radius in which the proximal radius is greater than the distal radius.

11. The robotic medical system of Claim 9, wherein the visual indicator comprises the non-ridged segment, optionally wherein an axial length of the first segment of ridges is greater than an axial length of the smooth segment.

12. The robotic medical system of Claim 9, further comprising a smooth segment on the outer surface proximal to the first segment of ridges.

13. The robotic medical system of Claim 9, wherein the visual indicator is a first visual indicator, and the cannula further comprises:
a second visual indicator on the tubular portion indicative of a second defined remote center of motion (410b) about which the tubular portion is pivotable by the robotic system;
a third segment (585c) of ridges on the outer surface of the tubular portion, wherein the third segment of ridges is disposed distal to the first visual indicator and proximal to the second visual indicator; and
a fourth segment (585d) of ridges on the outer surface of the tubular portion, wherein the fourth segment of ridges is disposed distal to the second visual indicator; and optionally further comprising a smooth segment (586b) on the outer surface axially interposed between the second and third segments of ridges.

## Patentansprüche

1. Medizinisches Robotersystem, umfassend:
eine Kanüle (460), die einen ersten definierten entfernten Mittelpunkt einer Bewegung (410a) und einen zweiten definierten entfernten Mittelpunkt einer Bewegung (410b) aufweist, der von dem ersten definierten entfernten Mittelpunkt axial beabstandet sind, wobei eine Außenoberfläche (481) der Kanüle Stabilitätsrippen (483) umfasst, die konfiguriert sind, um eine Gewebewand eines Patienten in Eingriff zu nehmen;
ein längliches medizinisches Instrument (350), das konfiguriert ist, um durch die Kanüle hindurch in den Patienten eingeführt zu werden;
einen Roboterarm (76, 82), der konfiguriert ist, um das längliche medizinische Instrument zu manipulieren, um einen medizinischen Eingriff durchzuführen; und
eine Steuervorrichtung (182), die konfiguriert ist, um die Bewegung des Roboterarms zu steuern, während die Bewegung um den ersten definierten entfernten Mittelpunkt herum beibehalten wird, und um von dem Beibehalten der Bewegung um den ersten definierten entfernten Mittelpunkt herum auf das Beibehalten der Bewegung um den zweiten definierten entfernten Mittelpunkt herum umzuschalten,
wobei ein erstes Segment (485) der Stabilitätsrippen auf einer ersten Seite des ersten definierten entfernten Mittelpunkts angeordnet ist und ein zweites Segment (485) der Stabilitätsrippen auf einer zweiten Seite des ersten definierten entfernten Mittelpunkts angeordnet ist, die von der ersten Seite des ersten definierten entfernten Mittelpunkts axial beabstandet ist, wobei das erste Segment der Stabilitätsrippen durch ein nicht geripptes Segment (486) der Kanüle von dem zweiten Segment der Stabilitätsrippen getrennt ist, wobei der erste definierte entfernte Mittelpunkt an dem nicht gerippten Segment positioniert ist, und wobei ein drittes Segment (585c) der Stabilitätsrippen auf einer ersten Seite des zweiten definierten entfernten Mittelpunkts angeordnet ist und ein viertes Segment (585d) der Stabilitätsrippen auf einer zweiten Seite des zweiten definierten entfernten Mittelpunkts angeordnet ist, die von der ersten Seite des zweiten definierten entfernten Mittelpunkts axial beabstandet ist.

2. Medizinisches Robotersystem nach Anspruch 1, wobei die Kanüle ferner eine Markierung an dem ersten definierten entfernten Mittelpunkt aufweist.

3. Medizinisches Robotersystem nach Anspruch 1, wobei:
der Roboterarm einen Instrumentenmanipulator (312) umfasst, der konfiguriert ist, um an dem länglichen medizinischen Instrument und an der Kanüle befestigt zu werden; und
die Steuervorrichtung konfiguriert ist, um die Bewegung des Roboterarms zu steuern, so dass jedes des länglichen medizinischen Instruments und der Kanüle um den ersten definierten entfernten Mittelpunkt herum schwenken.

4. Medizinisches Robotersystem nach Anspruch 1, wobei zwei oder mehr der Stabilitätsrippen konfiguriert sind, um unterschiedliche taktile Rückmeldungen bereitzustellen, wenn sie mit der Gewebewand des Patienten in Eingriff stehen.

5. Medizinisches Robotersystem nach Anspruch 1, wobei sich zwei oder mehr Sätze der Stabilitätsrippen mindestens in einem von Form, Größe oder Abstand voneinander unterscheiden.

6. Medizinisches Robotersystem nach Anspruch 1, wobei mindestens eine der Rippen konfiguriert ist, um der Bewegung gegen die Gewebewand in einer ersten axialen Richtung stärker zu widerstehen als die Rippe der Bewegung gegen die Gewebewand in einer zweiten axialen Richtung widersteht, die der ersten axialen Richtung entgegengesetzt ist.

7. Medizinisches Robotersystem nach Anspruch 1, wobei ein oberes Ende des ersten Segments der Stabilitätsrippen um einen festen Abstand von dem ersten definierten entfernten Mittelpunkt derart axial beabstandet ist, dass das obere Ende von außerhalb des Patienten sichtbar ist, wenn sich der erste definierte entfernte Mittelpunkt in einer geeigneten Einführtiefe in dem Patienten befindet, und das obere Ende in dem Patienten verdeckt ist, wenn sich der erste definierte entfernte Mittelpunkt tiefer als die geeignete Einführtiefe befindet.

8. Medizinisches Robotersystem nach Anspruch 1, wobei:
das zweite und das dritte Segment von Stabilitätsrippen durch einen nicht gerippten Abschnitt (586b) der Kanüle getrennt sind; und
ein oberes Ende des dritten Segments der Stabilitätsrippen von dem zweiten definierten entfernten Mittelpunkt um einen festen Abstand derart axial beabstandet ist, dass das obere Ende des dritten Segments von außerhalb des Patienten sichtbar ist, wenn sich der zweite definierte entfernte Mittelpunkt in einer geeigneten Einführtiefe im Patienten befindet, und das obere Ende des dritten Segments in dem Patienten verdeckt ist, wenn sich der zweite definierte entfernte Mittelpunkt tiefer als die geeignete Einführtiefe befindet.

9. Medizinisches Robotersystem nach Anspruch 1, wobei die Kanüle ferner umfasst:
einen Trichterabschnitt (462);
einen röhrenförmigen Abschnitt (480), der sich von dem Trichterabschnitt distal erstreckt;
einen visuellen Indikator (487) auf dem röhrenförmigen Abschnitt, der das erste definierte entfernte Bewegungszentrum indiziert; und
ein inneres Lumen (482), das sich durch den röhrenförmigen Abschnitt hindurch erstreckt,
wobei das erste Segment von Rippen proximal zu dem viusellen Indikator angeordnet ist, und
wobei das zweite Segment von Rippen zu dem visuellen Indikator distal angeordnet ist.

10. Medizinisches Robotersystem nach Anspruch 9, wobei die Rippen mindestens eine konische Rippe umfassen, die einen distalen Radius und einen proximalen Radius aufweist, wobei der proximale Radius größer als der distale Radius ist.

11. Medizinisches Robotersystem System nach Anspruch 9, wobei der visuelle Indikator das nicht gerippte Segment umfasst, optional wobei eine axiale Länge des ersten Segments von Rippen größer als eine axiale Länge des glatten Segments ist.

12. Medizinisches Robotersystem System nach Anspruch 9, ferner umfassend ein glattes Segment auf der Außenoberfläche proximal zu dem ersten Segment von Rippen.

13. Medizinisches Robotersystem nach Anspruch 9, wobei der visuelle Indikator ein erster visueller Indikator ist und die Kanüle ferner umfasst:
einen zweiten visuellen Indikator auf dem röhrenförmigen Abschnitt, der einen zweiten definierten entfernten Mittelpunkt der Bewegung (410b) indiziert, um den herum der röhrenförmige Abschnitt durch das Robotersystem schwenkbar ist;
ein drittes Segment (585c) von Rippen auf der Außenoberfläche des röhrenförmigen Abschnitts, wobei das dritte Segment von Rippen distal zu dem ersten visuellen Indikator und proximal zu dem zweiten visuellen Indikator angeordnet ist; und
ein viertes Segment (585d) von Rippen auf der Außenoberfläche des röhrenförmigen Abschnitts, wobei das vierte Segment von Rippen zu dem zweiten visuellen Indikator distal angeordnet ist;
und optional ferner umfassend ein glattes Segment (586b) auf der Außenoberfläche, das zwischen dem zweiten und dem dritten Segment von Rippen axial angeordnet ist.

## Revendications

1. Système médical robotique, comprenant :
une canule (460) présentant un premier centre de mouvement à distance défini (410a) et un second centre de mouvement à distance défini (410b) espacé de manière axiale du premier centre à distance défini, dans lequel une surface extérieure (481) de la canule comprend des crêtes de stabilité (483) conçues pour entrer en prise dans une paroi tissulaire d'un patient ;
un instrument médical allongé (350) conçu pour être inséré dans le patient à travers la canule ;
un bras robotique (76, 82) configuré pour manipuler l'instrument médical allongé afin de réaliser un acte médical ; et
un dispositif de commande (182) configuré pour commander le mouvement du bras robotique tout en maintenant le mouvement autour du premier centre à distance défini et pour passer du maintien du mouvement autour du premier centre à distance défini au maintien du mouvement autour du second centre à distance défini,
dans lequel un premier segment (485) des crêtes de stabilité est disposé sur un premier côté du premier centre à distance défini et un deuxième segment (485) des crêtes de stabilité est disposé sur un second côté du premier centre à distance défini, espacé de manière axiale du premier côté du premier centre à distance défini, dans lequel le premier segment des crêtes de stabilité est séparé du deuxième segment des crêtes de stabilité par un segment non strié (486) de la canule, dans lequel le premier centre à distance défini est positionné au niveau du segment non strié, et dans lequel un troisième segment (585c) des crêtes de stabilité est disposé sur un premier côté du second centre à distance défini et un quatrième segment (585d) des crêtes de stabilité est disposé sur un second côté du second centre à distance défini, espacé de manière axiale du premier côté du second centre à distance défini.

2. Système médical robotique selon la revendication 1, dans lequel la canule comprend en outre un marquage au niveau du premier centre à distance défini.

3. Système médical robotique selon la revendication 1, dans lequel :
le bras robotique comprend un manipulateur d'instruments (312) configuré pour se fixer à l'instrument médical allongé et à la canule ; et
le dispositif de commande est configuré pour commander le mouvement du bras robotique de telle sorte que l'instrument médical allongé et la canule pivotent autour du premier centre à distance défini.

4. Système médical robotique selon la revendication 1, dans lequel deux ou plusieurs des crêtes de stabilité sont conçues pour fournir une rétroaction tactile différente l'une de l'autre lorsqu'elles sont en prise avec la paroi tissulaire du patient.

5. Système médical robotique selon la revendication 1, dans lequel deux ou plusieurs ensembles de crêtes de stabilité diffèrent l'un de l'autre dans au moins une parmi une forme, une taille ou un pas.

6. Système médical robotique selon la revendication 1, dans lequel au moins une des crêtes est conçue pour résister au mouvement contre la paroi tissulaire dans une première direction axiale plus grande que la crête résiste au mouvement contre la paroi tissulaire dans une seconde direction axiale opposée à la première direction axiale.

7. Système médical robotique selon la revendication 1, dans lequel une extrémité supérieure du premier segment des crêtes de stabilité est espacée de manière axiale du premier centre à distance défini d'une distance fixe de telle sorte que, lorsque le premier centre à distance défini est à une profondeur d'insertion appropriée dans le patient, l'extrémité supérieure est visible de l'extérieur du patient, et, lorsque le premier centre à distance défini est plus profond que la profondeur d'insertion appropriée, l'extrémité supérieure est masquée à l'intérieur du patient.

8. Système médical robotique selon la revendication 1, dans lequel :
les deuxième et troisième segments des crêtes de stabilité sont séparés par une partie non striée (586b) de la canule ; et
une extrémité supérieure du troisième segment des crêtes de stabilité est espacée de manière axiale du second centre à distance défini d'une distance fixe de telle sorte que, lorsque le second centre à distance défini est à une profondeur d'insertion appropriée dans le patient, l'extrémité supérieure du troisième segment est visible de l'extérieur du patient et, lorsque le second centre à distance défini est plus profond que la profondeur d'insertion appropriée, l'extrémité supérieure du troisième segment est masquée à l'intérieur du patient.

9. Système médical robotique selon la revendication 1, dans lequel la canule comprend en outre :
une partie en forme d'entonnoir (462) ;
une partie tubulaire (480) s'étendant de manière distale à partir de la partie en forme d'entonnoir ;
un indicateur visuel (487) sur la partie tubulaire indiquant le premier centre de mouvement à distance défini ; et
une lumière interne (482) s'étendant à travers la partie tubulaire,
dans lequel le premier segment de crêtes est disposé de manière proximale par rapport à l'indicateur visuel, et
dans lequel le second segment de crêtes est disposé de manière distale par rapport à l'indicateur visuel.

10. Système médical robotique selon la revendication 9, dans lequel les crêtes comprennent au moins une crête conique présentant un rayon distal et un rayon proximal dans lequel le rayon proximal est plus grand que le rayon distal.

11. Système médical robotique selon la revendication 9, dans lequel l'indicateur visuel comprend le segment non strié, éventuellement dans lequel une longueur axiale du premier segment de crêtes est supérieure à une longueur axiale du segment lisse.

12. Système médical robotique selon la revendication 9, comprenant en outre un segment lisse sur la surface extérieure proximale du premier segment de crêtes.

13. Système médical robotique selon la revendication 9, dans lequel l'indicateur visuel est un premier indicateur visuel, et la canule comprend en outre :
un second indicateur visuel sur la partie tubulaire indiquant un second centre de mouvement à distance défini (410b) autour duquel la partie tubulaire peut être pivotée par le système robotique ;
un troisième segment (585c) de crêtes sur la surface extérieure de la partie tubulaire, dans lequel le troisième segment de crêtes est disposé de manière distale par rapport au premier indicateur visuel et de manière proximale par rapport au second indicateur visuel ; et
un quatrième segment (585d) de crêtes sur la surface extérieure de la partie tubulaire, le quatrième segment de crêtes étant disposé à distance du second indicateur visuel ;
et comprenant en outre éventuellement un segment lisse (586b) sur la surface externe, de manière axiale interposé entre les deuxième et troisième segments de crêtes.
